# EUROPEAN PATENT SPECIFICATION

(11) **EP 3 976 029 B1**
(45) Date of publication and mention of the grant of the patent: **11.11.2026**
(21) Application number: 20812948.6
(22) Date of filing: 22.05.2020
(51) Int. Cl.: C07D 271/12, C07D 285/14, C07D 417/14, A61K 31/4245

(54) **ASPARAGINE ENDOPEPTIDASE (AEP) INHIBITORS, COMPOSITIONS, AND USES RELATED THERETO**
ASPARAGIN-ENDOPEPTIDASE(AEP)-INHIBITOREN, ZUSAMMENSETZUNGEN UND ZUGEHÖRIGE VERWENDUNGEN
INHIBITEURS D'ASPARAGINE ENDOPEPTIDASE (AEP), COMPOSITIONS ET UTILISATIONS ASSOCIÉES

(30) Priority: 24.05.2019 US 201962852548 P
(43) Date of publication of application: 06.04.2022
(73) Proprietor: Emory University, Atlanta, Georgia 30322 (US)
(72) Inventor: YE, Keqiang, Atlanta, Georgia 30322 (US)
(74) Representative: Angerhausen, Christoph
(86) International application number: PCT/US2020/034193
(87) International publication number: WO 2020/242933

(56) References cited:
- US-A1- 2010 119 475
- US-A1- 2010 184 601
- US-A1- 2017 029 388
- US-A1- 2017 166 569
- EDUARDO H. G. DA CRUZ ET AL: "Design, synthesis and application of fluorescent 2,1,3-benzothiadiazole-triazole-linked biologically active lapachone derivatives", NEW JOURNAL OF CHEMISTRY, vol. 38, no. 6, 1 January 2014 (2014-01-01), GB, pages 2569, XP055549064, ISSN: 1144-0546, DOI: 10.1039/c3nj01499a

## Description

### BACKGROUND

Asparagine endopeptidase (AEP), also known as legumain, is a lysosomal cysteine protease that cleaves peptide bonds C-terminally to asparagine residues. AEP is involved in various cellular events, including antigen processing, the cleavage of other lysosomal enzymes, and osteoclast formation. In mammals, AEP is highly expressed in the kidneys; mice deficient in AEP accumulate various proteins in the endosomes and lysosomes of the proximal tubule cells, which results in a pathology consisting of hyperplasia, fibrosis and glomerular cysts. AEP-null mice exhibit symptoms similar to those of hemophagocytic lymphohistiocytosis, suggesting the enzyme is involved in the pathophysiology of this disease. Biochemically, the enzyme is highly regulated by its specificity for asparagine residues and pH. Dysregulated AEP activity has been implicated in various diseases, including cancers and neurodegeneration.

AEP activates MMP-2 (Progelatinase A) by proteolytic removal of an N-terminal propeptide. While only a limited quantity of AEP is detected in normal tissues, the enzyme is overexpressed on the cell surface and in cytoplasmic vesicles of solid tumors. The endoprotease activity of AEP has been associated with increased invasive and aggressive behavior of several cancers, including breast, prostate, colorectal and gastric carcinomas. See WO2015157376; Gawenda et al., Legumain expression as a prognostic factor in breast cancer patients, Breast Cancer Res Treat, 2007, 102, 1-6; Ohno et al., Association of legumain expression pattern with prostate cancer invasiveness and aggressiveness, World J Urol, 2013, 31, 359-364; Li et al., Effects of legumain as a potential prognostic factor on gastric cancers, Med Oncol, 2013, 30, 621; and Haugen et al., Nuclear legumain activity in colorectal cancer, PLoS One, 2013, 8, e52980.

Zhang et al. report asparagine endopeptidase is a therapeutic target for neurodegenerative diseases. Expert Opin Ther Targets, 2016, 20(10):1237-45. See also WO2015157382.

Basurto-Islas et al., report activation of asparaginyl endopeptidase leads to tau hyperphosphorylation in Alzheimer disease, J Biol Chem, 288, 2013, 17495-17507. See also Chan et al., Mice lacking asparaginyl endopeptidase develop disorders resembling hemophagocytic syndrome, Proc Natl Acad Sci USA, 2009, 106, 468-473 and Herskowitz et al., Asparaginyl endopeptidase cleaves TDP-43 in brain, Proteomics, 2012, 12, 2455-2463.

US 2017/029 388 A1 relates to asparagine endopeptidase inhibitors and compositions thereof.

US 2017/166 569 A1 relates to asparagine endopeptidase inhibitors for managing cancer and compositions related thereto. The asparagine endopeptidase inhibitors are substituted 3,7-dihydropurine-2,6-dione derivatives. The pharmaceutical compositions comprising an asparagine endopeptidase inhibitor may further comprise a pharmaceutically acceptable excipient.

References cited herein are not an admission of prior art.

### SUMMARY

The present invention relates to compounds of formula IIB as defined herein, which are asparagine endopeptidase inhibitors. The invention also provides pharmaceutical compositions comprising these compounds and their use in the treatment of diseases. The invention is further set out in the appended claims. In certain embodiments, the asparagine endopeptidase inhibitors are useful for improving memory, treating or preventing cancer, neurodegenerative diseases and cognitive disorders. In certain embodiments, the disclosure relates to pharmaceutical compositions comprising an asparagine endopeptidase inhibitor disclosed herein and a pharmaceutically acceptable excipient. In certain embodiments, the disclosure relates to the compounds of formula IIB for use in the treatment of a cancer or neurodegenerative disease comprising administering an effective amount of pharmaceutical composition an asparagine endopeptidase inhibitor disclosed herein to a subject in need thereof.

The present invention relates to compounds having the following Formula IIB: 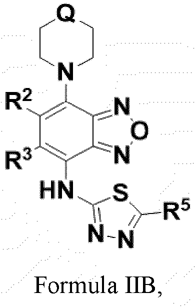 or salts thereof wherein, the substituents are described herein.

In certain embodiments, this disclosure relates to pharmaceutical compositions comprising compounds as reported herein and a pharmaceutically acceptable excipient. In certain embodiments, the pharmaceutical composition is in the form of a tablet, pill, capsule, gel, gel capsule, or cream. In certain embodiments, the pharmaceutical composition is in the form of a sterilized pH buffered aqueous salt solution or a saline phosphate buffer between a pH of 6 to 10 or an isotonic phosphate buffered saline solution, optionally comprising a saccharide or polysaccharide. In certain embodiments, the pharmaceutical composition is in solid form surrounded by an enteric coating. In certain embodiments, the enteric coatings comprise methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, or combinations thereof.

In certain embodiments, the pharmaceutically acceptable excipient is selected from lactose, sucrose, mannitol, triethyl citrate, dextrose, cellulose, methyl cellulose, ethyl cellulose, hydroxyl propyl cellulose, hydroxypropyl methylcellulose, carboxymethylcellulose, croscarmellose, sodium, polyvinyl N-pyrrolidone, crospovidone, ethyl cellulose, povidone, methyl and ethyl acrylate copolymer, polyethylene glycol, fatty acid esters of sorbitol, lauryl sulfate, gelatin, glycerin, glyceryl monooleate, silicon dioxide, titanium dioxide, talc, corn starch, carnauba wax, stearic acid, sorbic acid, magnesium stearate, calcium stearate, castor oil, mineral oil, calcium phosphate, starch, carboxymethyl ether of starch, iron oxide, triacetin, acacia gum, esters, or salts thereof.

In certain embodiments, this disclosure relates to the compound according to the present invention for use in treating cancer comprising administering an effective amound of a compound as reported herein to a subject in need thereof. In certain embodiments, the subject is at risk of, exhibiting symptoms, of or diagnosed with cancer.

In certain embodiments, this disclosure relates to of the compound according to the present invention for use in treating or preventing a congative disorder or memory loss comprising administering an effective amound of a compound as reported herein to a subject in need thereof. In certain embodiments, the subject is at risk of, exhibiting symptoms, of or diagnosed with a cognative disorder.

In certain embodiments, this disclosure relates to of the compound according to the present inventin for use in improving memory comprising administering an effective amound of a compound as reported herein to a subject in need thereof. In certain embodiments, the subject is at risk of, exhibiting symptoms, of or diagnosed with a risk or developing a congnative disorder. In certain embodiments, the congative disorder is Alzheimer's, Parkinson's, Huntington's, Multiple Sclerosis, or ALS.

In certain embodiments, this disclosure relates to methods of producing compound as reported herein comprising mixing starting materials with reagents under conditions such that the compounds are formed.

### BRIEF DESCRIPTION OF THE DRAWINGS

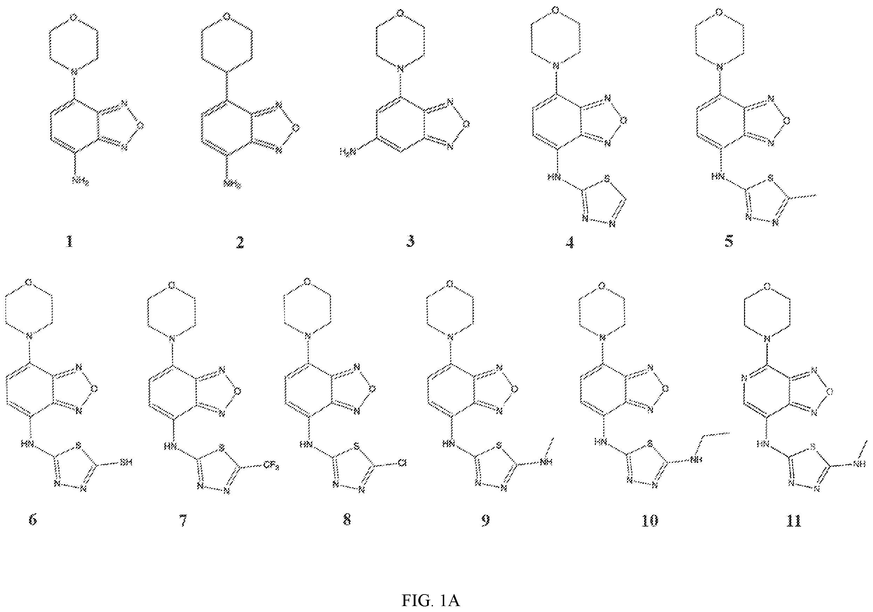

Figure 1A illustrates (reference) compounds of this disclosure.

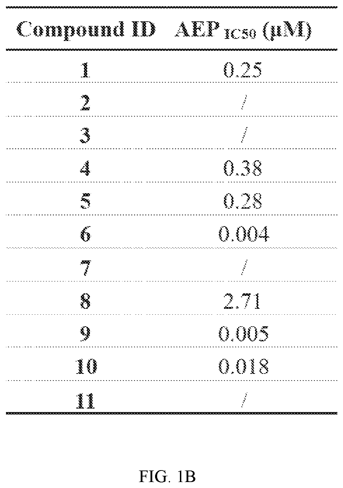

Figure 1B show data for compound illustrated in Fig. 1A.

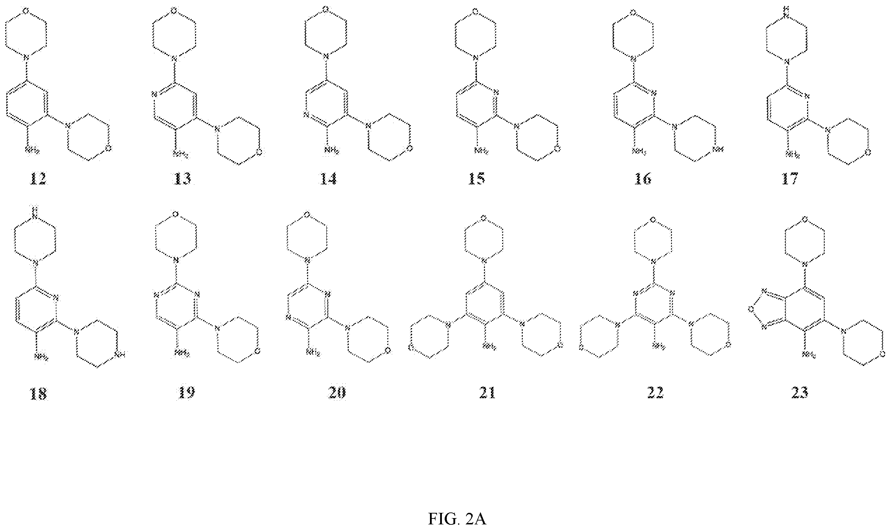

Figure 2A illustrates reference compounds of this disclosure.

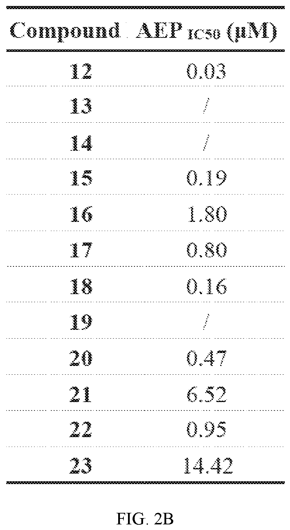

Figure 2B show data for compound illustrated in Fig. 2A.

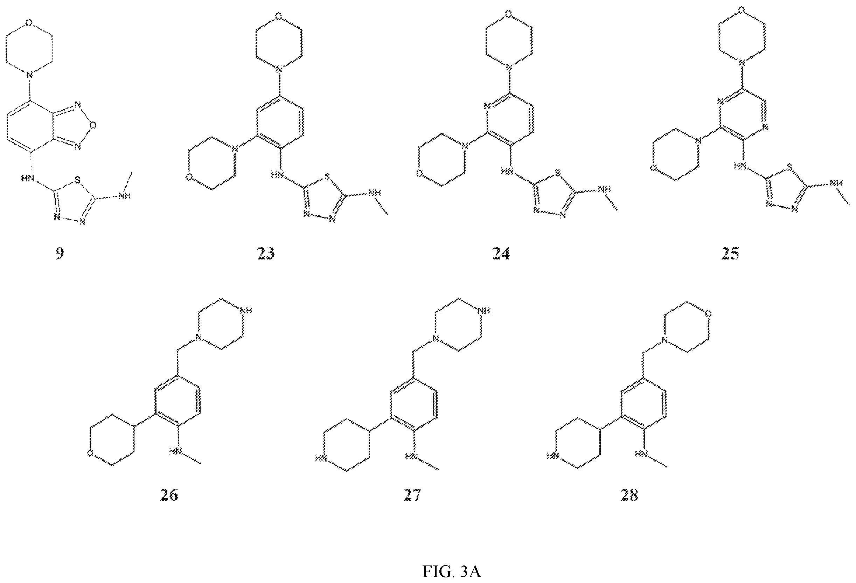

Figure 3A illustrates (reference) compounds of this disclosure.

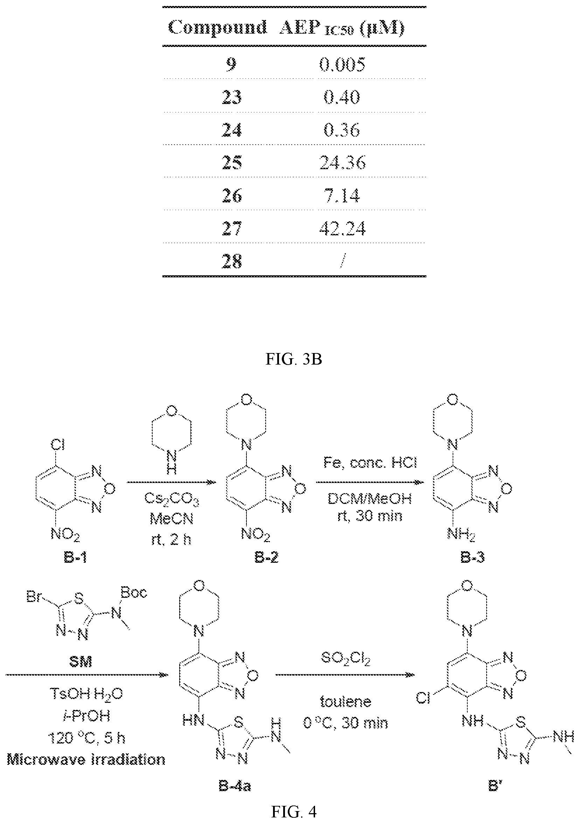

Figure 3B show data for compound illustrated in Fig. 3A.
Figure 4 illustrates the preparation of N²-(5-chloro-7-morpholinobenzo[c][1,2,5]oxadiazol-4-yl)-N⁵-methyl-1,3,4-thiadiazole-2,5-diamine.

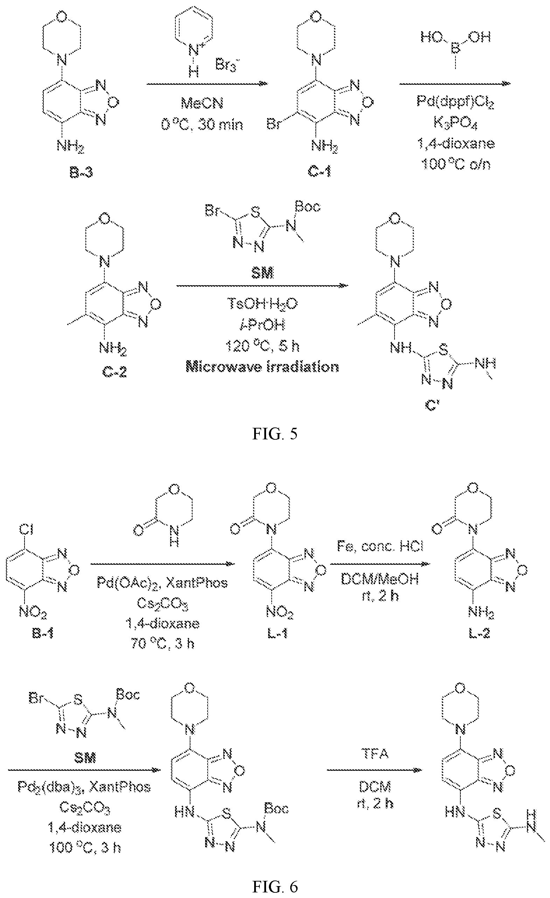

Figure 5 illustrates the preparation of N²-methyl-N⁵-(5-methyl-7-morpholinobenzo[c][1,2,5]oxadiazol-4-yl)-1,3,4-thiadiazole-2,5-diamine.
Figure 6 illustrates the synthesis of N²-methyl-N⁵-(7-morpholinobenzo[c][1,2,5]oxadiazol-4-yl)-1,3,4-thiadiazole-2,5-diamine.

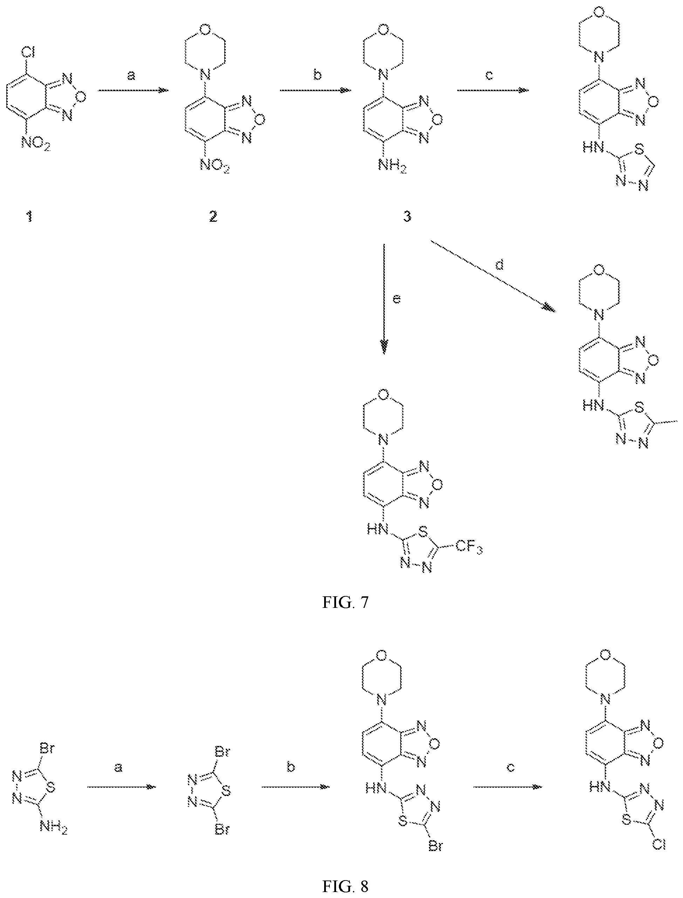

Figure 7 illustrates the synthesis of (reference) embodiments of this disclosure. Reagents and conditions: (a) morpholine, K₃PO₄, room temperature, overnight; (b) conc. HCl, Fe, room temperature, overnight; (c) 2-bromo-[1,3,4]thiadiazole, PTSA, isopropanol, 80 °C, overnight; (d) 2-bromo-5-methyl-[1,3,4]thiadiazole, PTSA, isopropanol, 105 °C, 24 h; (e) 2-chloro-5-trifluoromethyl-[1,3,4]thiadiazole, PTSA, isopropanol, 105 °C, 24 h.
Figure 8 illustrates the synthesis of reference embodiments of this disclosure. Reagents and conditions: (a) HBr/Br₂/NaNO₂, 0 °C, overnight; (b) compound 3/ PTSA/ isopropanol, 100 °C,24 h; (c) conc. HCl, 60 °C, 2 days.

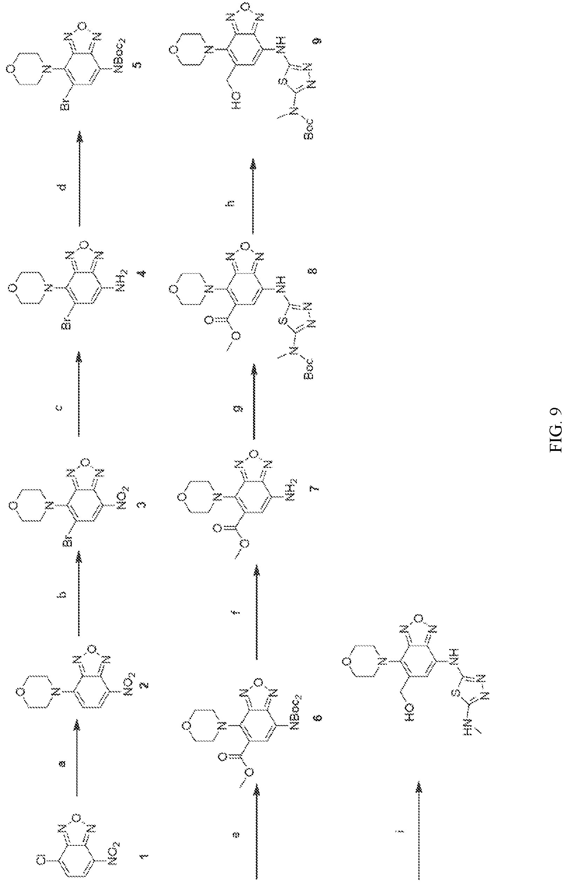

Figure 9 illustrates the synthesis of embodiments of this disclosure. Reagents and conditions: (a) morpholine, K₂CO₃, EtOH, rt; (b) NBS, CH₃CN, 60 °C; (c) Fe, HCl, MeOH, rt; (d) Boc₂O, DMAP, THF, rt; (e) CO, TEA, MeOH, Pd(dppf)Cl₂, 85 °C ; (f) TFA, DCM, rt; (g) tert-butyl (5-bromo-1,3,4-thiadiazol-2-yl)(methyl)carbamate, Pd₂(dba)₃, Xantphos, Cs₂CO₃, dioxane, 100 °C; (h) DIBAL-H, DCM, -78 °C; (i) TFA, DCM, rt.

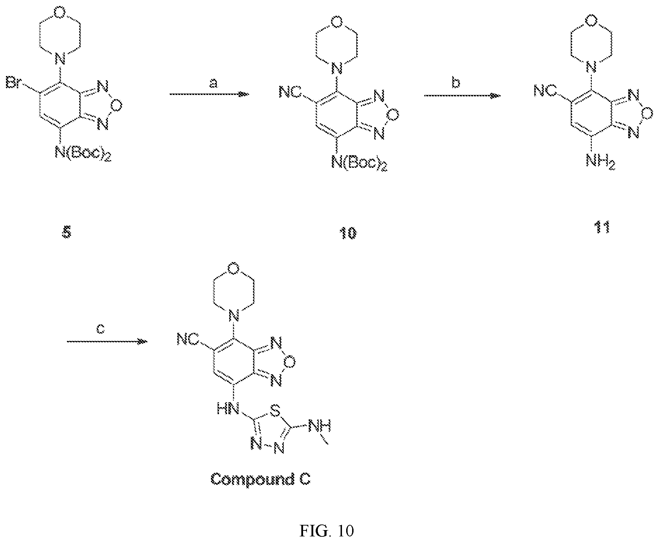

Figure 10 illustrates the synthesis of embodiments of this disclosure. Reagents and conditions: (a) Zn(CN)₂, dppf, Pd₂(dba)₃, NMP, 100 °C; (b) TFA, DCM, rt; (c) 5-bromo-N-methyl-1,3,4-thiadiazol-2-amine, PTSA, NMP, M.W., 150 °C.

### DETAILED DISCUSSION

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments. Any recited method can be carried out in the order of events recited or in any other order that is logically possible.

Embodiments of the present disclosure will employ, unless otherwise indicated, techniques of medicine, organic chemistry, biochemistry, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise.

As used herein, the term "combination with" when used to describe administration with an additional treatment means that the agent may be administered prior to, together with, or after the additional treatment, or a combination thereof.

As used herein, the term "derivative" refers to a structurally similar compound that retains sufficient functional attributes of the identified analogue. The derivative may be structurally similar because it is lacking one or more atoms, substituted, a salt, in different hydration/oxidation states, or because one or more atoms within the molecule are switched, such as, but not limited to, replacing an oxygen atom with a sulfur atom or replacing an amino group with a hydroxy group. Contemplated derivatives include switching carbocyclic, aromatic or phenyl rings with heterocyclic rings or switching heterocyclic rings with carbocyclic, aromatic or phenyl rings, typically of the same ring size. Derivatives may be prepare by any variety of synthetic methods or appropriate adaptations presented in synthetic or organic chemistry text books, such as those provide in March's Advanced Organic Chemistry: Reactions, Mechanisms, and Structure, Wiley, 6th Edition (2007) Michael B. Smith or Domino Reactions in Organic Synthesis, Wiley (2006) Lutz F. Tietze.

The term "substituted" refers to a molecule wherein at least one hydrogen atom is replaced with a substituent. When substituted, one or more of the groups are "substituents." The molecule may be multiply substituted. In the case of an oxo substituent ("=O"), two hydrogen atoms are replaced. Example substituents within this context may include halogen, hydroxy, alkyl, alkoxy, nitro, cyano, oxo, carbocyclyl, carbocycloalkyl, heterocarbocyclyl, heterocarbocycloalkyl, aryl, arylalkyl, heteroaryl, heteroarylalkyl, -NRaRb, -NRaC(=O)Rb, -NRaC(=O)NRaNRb, - NRaC(=O)ORb, - NRaSO₂Rb, -C(=O)Ra, -C(=O)ORa, -C(=O)NRaRb, -OC(=O)NRaRb, -ORa, - SRa, -SORa, -S(=O)₂Ra, -OS(=O)₂Ra and -S(=O)₂ORa. Ra and Rb in this context may be the same or different and independently hydrogen, halogen hydroxy, alkyl, alkoxy, alkyl, amino, alkylamino, dialkylamino, carbocyclyl, carbocycloalkyl, heterocarbocyclyl, heterocarbocycloalkyl, aryl, arylalkyl, heteroaryl, heteroarylalkyl.

As used herein, "alkyl" means a noncyclic straight chain or branched, unsaturated or saturated hydrocarbon such as those containing from 1 to 10 carbon atoms. Representative saturated straight chain alkyls include methyl, ethyl, n-propyl, n-butyl, n-pentyl, n-hexyl, n-septyl, n-octyl, n-nonyl, and the like; while saturated branched alkyls include isopropyl, sec-butyl, isobutyl, tert-butyl, isopentyl, and the like. Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3 -methyl- 1-butenyl, 2-methyl-2-butenyl, 2,3-dimethyl-2-butenyl, and the like; while representative straight chain and branched alkynyls include acetylenyl, propynyl, 1-butynyl, 2-butynyl, 1-pentynyl, 2-pentynyl, 3- methyl-1-butynyl, and the like.

Non-aromatic mono or polycyclic alkyls are referred to herein as "carbocycles" or "carbocyclyl" groups. Representative saturated carbocycles include cyclopropyl, cyclobutyl, cyclopentyl, cyclohexyl, and the like; while unsaturated carbocycles include cyclopentenyl and cyclohexenyl, and the like.

"Heterocarbocycles" or heterocarbocyclyl" groups are carbocycles which contain from 1 to 4 heteroatoms independently selected from nitrogen, oxygen and sulfur which may be saturated or unsaturated (but not aromatic), monocyclic or polycyclic, and wherein the nitrogen and sulfur heteroatoms may be optionally oxidized, and the nitrogen heteroatom may be optionally quaternized. Heterocarbocycles include morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

"Aryl" means an aromatic carbocyclic monocyclic or polycyclic ring such as phenyl or naphthyl. Polycyclic ring systems may, but are not required to, contain one or more non-aromatic rings, as long as one of the rings is aromatic.

As used herein, "heteroaryl" or "heteroaromatic" refers an aromatic heterocarbocycle having 1 to 4 heteroatoms selected from nitrogen, oxygen and sulfur, and containing at least 1 carbon atom, including both mono- and polycyclic ring systems. Polycyclic ring systems may, but are not required to, contain one or more non-aromatic rings, as long as one of the rings is aromatic. Representative heteroaryls are furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, and quinazolinyl. It is contemplated that the use of the term "heteroaryl" includes N-alkylated derivatives such as a 1-methylimidazol-5-yl substituent.

As used herein, "heterocycle" or "heterocyclyl" refers to mono- and polycyclic ring systems having 1 to 4 heteroatoms selected from nitrogen, oxygen and sulfur, and containing at least 1 carbon atom. The mono- and polycyclic ring systems may be aromatic, non-aromatic or mixtures of aromatic and non-aromatic rings. Heterocycle includes heterocarbocycles, heteroaryls, and the like.

"Alkylthio" refers to an alkyl group as defined above attached through a sulfur bridge. An example of an alkylthio is methylthio, (i.e., -S-CH₃).

"Alkoxy" refers to an alkyl group as defined above attached through an oxygen bridge. Examples of alkoxy include, but are not limited to, methoxy, ethoxy, n-propoxy, i-propoxy, n-butoxy, s-butoxy, t-butoxy, n- pentoxy, and s-pentoxy. Preferred alkoxy groups are methoxy, ethoxy, n-propoxy, i- propoxy, n-butoxy, s-butoxy, t-butoxy.

"Alkylamino" refers an alkyl group as defined above attached through an amino bridge. An example of an alkylamino is methylamino, (i.e., -NH-CH₃).

"Alkanoyl" refers to an alkyl as defined above attached through a carbonyl bridge (i.e., - (C=O)alkyl).

"Alkylsulfonyl" refers to an alkyl as defined above attached through a sulfonyl bridge (i.e., -S(=O)₂alkyl) such as mesyl and the like, and "Arylsulfonyl" refers to an aryl attached through a sulfonyl bridge (i.e., - S(=O)₂aryl).

"Alkylsulfamoyl" refers to an alkyl as defined above attached through a sulfamoyl bridge (i.e., -S(=O)₂NHalkyl), and an "Arylsulfamoyl" refers to an alkyl attached through a sulfamoyl bridge (i.e., - S(=O)₂NHaryl).

"Alkylsulfinyl" refers to an alkyl as defined above with the indicated number of carbon atoms attached through a sulfinyl bridge (i.e. -S(=O)alkyl).

The terms "halogen" and "halo" refer to fluorine, chlorine, bromine, and iodine.

The term "aroyl" refers to an aryl group (which may be optionally substituted as described above) linked to a carbonyl group (e.g., -C(O)-aryl).

The term "sulfamoyl" refers to the amide of sulfonic acid (i.e., -S(=O)₂NRR')

Throughout the specification, groups and substituents thereof may be chosen to provide stable moieties and compounds.

### Blockade of Asparagine Endopeptidase Inhibits Cancer Metastasis

The aberrant expression of AEP in cancer cells and on the surface of tumor-associated macrophages has been linked to the enzyme's involvement in tumor development and metastasis. Lin et al. report selective ablation of tumor-associated macrophages suppresses metastasis and angiogenesis. Cancer Sci, 2013, 104, 1217-1225. There is evidence suggesting that AEP is a viable drug target and a biomarker for the diagnosis and progression of various cancers. Recent studies suggest that legumain expression could be a prognostic factor in patients with colorectal cancer, breast cancer, and ovarian cancer as well as a potential target for tumor therapy.

Peptide-based AEP inactivators may be conjugated to a nanoparticle and optionally another anti-cancer compound, e.g., doxorubicin, to target cancer cells, mitigating systemic toxicity. The AEP inhibitor may be used as a targeting molecule to direct the cancer drug specifically to cancer cells by exploiting the fact that AEP is extracellularly expressed on tumors and in tumor microenvironments. Liu et al., Targeting cell surface alpha(v)beta(3) integrin increases therapeutic efficacies of a legumain protease-activated auristatin prodrug, 2012, Mol Pharm 9, 168-175 and Liao et al., Synthetic enzyme inhibitor: a novel targeting ligand for nanotherapeutic drug delivery inhibiting tumor growth without systemic toxicity, Nanomedicine, 2011, 7, 665-673.

Although it is not intended that certain embodiments of this disclosure be limited by any particular mechanism, AEP inhibitors are likely imparting its effects by inhibiting the cleavage and activation of MMP-2. The matrix metalloproteinase is a known substrate of AEP, which cleaves a propeptide from the N-terminus of MMP-2, thus enabling the enzyme to degrade the extracellular matrix and promote more aggressive and invasive tumor growth. There is an overexpression of MMPs in the majority of human cancers, which is associated with an increase in invasive and metastatic behavior and an overall poor prognosis, since patients overexpressing these enzymes tend to have shorter survival rates. Additionally, in gastric cancer, the enhanced expression of MMP-2 has been most strongly correlated with a poor prognosis in comparison to any of the other MMPs.

MMP-2 cleavage in MDA-MB-231 cells and mammary tissue was inhibited by an AEP inhibitor in a dose-dependent manner, suggesting that the AEP inhibitor successfully regulates the activity of MMP-2. Legumain could degrade fibronectin, the main component of extracellular matrix. Conceivably, inhibition of AEP by inhibitors may potently block the breast-to-lung metastasis in mice. Therefore, an approach to prevent breast tumor metastasis is through the attenuation of MMP-2 activity by precluding its activation through the inhibition of AEP.

### Inhibition of Asparagine Endopeptidase is Neuroprotective and Improves Cognitive Memory

Ageing is the greatest risk factor for Alzheimer's disease (AD). During ageing, the pH in brain gradually decreases. AEP is progressively upregulated in mouse brain and activated in aged mice. Moreover, AEP is also elevated and activated in human AD brains compared to normal controls. The active AEP cleaves both APP (amyloid precursor protein) and Tau, two major pathogenic players in AD. AEP processing APP facilitates BACE1 to degrade APP, leading to β-amyloid upregulation. Knockout of AEP from AD transgenic mouse models reverses the pathological events in 5XFAD and APP/PS1 mice and improves the cognitive deficit. On the other hand, active AEP proteolytically degrades tau, abolishes its microtubule assembly function, induces tau aggregation, and triggers neurodegeneration. Furthermore, AEP is activated in tau P301s transgenic mice and human AD brain, leading to tau truncation in NFTs. Deletion of AEP from tau P301S transgenic mice substantially reduces NFTs deposit, alleviates the synapse loss and rescues impaired hippocampal synaptic plasticity and the cognitive deficits. AEP is primarily responsible for the hyperphosphorylation of tau through its cleavage of SET, a PP2A inhibitor after cleavage, which results in the inhibition of the enzyme responsible for 70% of tau phosphatase activity, Protein Phosphatase-2A (PP2A). AEP acts as a mediator in the onset and progression of AD. Inhibition of AEP can be a therapeutically useful for treating the neurodegenerative diseases including AD.

AEP is upregulated and activated in aged normal brain and human Alzheimer's Disease (AD) brain, playing a critical role in mediating the pathphysiology of AD. Disclosed herein are brain permeable and orally bioactive AEP inhibitor that reduces the senile plaque formation in AD mouse model and alleviates the cognitive defects. A high through-put screening was performed. Several skeletal families of compounds were discovered with potent inhibitory activities. A nontoxic and specific AEP inhibitor that was identified that selectively blocks AEP but not other related-cysteine proteases. Chronic treatment of 5XFAD mice with oral administration of the inhibitor ameliorates synapse loss and augments long-term potentiation (LTP), resulting in protection of memory loss in AD. Therefore, these findings indicate that these AEP inhibitors can be effective clinical therapeutic agents.

Stroke, seizures, and head trauma are all causative of brain tissue ischemia, which upregulates apoptotic and necrotic processes in brain tissue, implicating them as leading causes of neurodegeneration in humans. Depriving the brain of its blood supply induces an excitotoxic effect that causes neuronal death through an incompletely understood mechanism. A predominant feature of excitotoxicity is acidosis, which is a shift in the buffered brain interstitial pH from 7.3 to 6.0, resulting from increased cellular concentrations of the excitatory amino acid, glutamate. In response to the decrease in intracranial pH, caused by excitatory acidosis, AEP is activated and has been shown to display aberrant activity toward one of its substrates, SET, a DNAse inhibitor. SET is a phosphoprotein and is predominantly localized to the nucleus, where it is involved in transcriptional regulation through interactions with histone tails. SET also acts as a mediator of apoptosis, by inhibiting DNA nicking, in the Granzyme-A-mediated cell death pathway. AEP is activated following induction of ischemia and acidosis, and proteolytically cleaves SET, which results in neuronal cell death; whereas, SET remains intact in AEP-deficient mice and neuronal cell death is negligible. This observation suggests that AEP inhibition provides a way to prevent neurodegeneration following stroke, seizure or head trauma.

AEP is primarily responsible for the hyperphosphorylation of tau through its cleavage of SET, which results in the inhibition of the enzyme responsible for 70% of tau phosphatase activity, Protein Phosphatase-2A (PP2A). The levels of active AEP and cleaved N-terminal and C-terminal fragments of SET are elevated in the brains of AD patients; additionally, acidosis was found to trigger the cytoplasmic translocation of AEP and SET from the lysosome and nucleus, respectively. This finding indicates that AEP seems to play a role in the etiopathogenesis of Alzheimer's Disease.

AD is the most common neurodegenerative diseases. It is characterized by the deposition of Aβ and insoluble tau. AEP cleaves APP and tau in the AD brain. Compared to the full-length APP, the AEP-generated APP fragment is a better substrate for β-secretase, thus enhance the production of Aβ. Tau cleavage by AEP will generate several fragments that can promote it deposition. Furthermore, cleavage of SET by AEP promotes neuronal death induced by ischemia, and promotes the phosphorylation of tau. All these observations indicate AEP inhibitors may rescue the progressive neurodegeneration in AD.

### Asparagine endopeptidase inhibitors

This disclosure relates to asparagine endopeptidase inhibitors. The asparagine endopeptidase inhibitor is a substituted benzo[c][1,2,5]oxadiazole derivative.

The present invention relates to compounds of the following formula IIB: 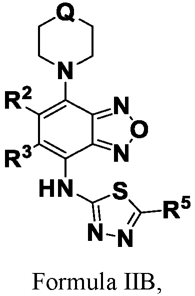
or salts thereof, wherein
Q is O;
R² is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R² is optionally substituted with one or more, the same or different, R²⁰;
R²⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R²⁰ is optionally substituted with one or more, the same or different, R²¹;
R²¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl;
R³ is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³ is optionally substituted with one or more, the same or different, R³⁰;
R³⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³⁰ is optionally substituted with one or more, the same or different, R³¹;
R³¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl;
R⁵ is selected from amino, mercapto and alkylamino

In certain embodiments, this disclosure relates to compounds of the following formula IIC: 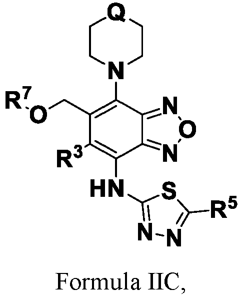
prodrugs, esters, derivatives, or salts thereof wherein,
Q is O;
R³ is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³ is optionally substituted with one or more, the same or different, R³⁰;
R³⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³⁰ is optionally substituted with one or more, the same or different, R³¹;
R³¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl;
R⁵ is selected from amino, mercapto, and alkylamino;
R⁷ is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R⁷ is optionally substituted with one or more, the same or different, R⁷⁰;
R⁷⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R⁷⁰ is optionally substituted with one or more, the same or different, R⁷¹; and
R⁷¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl.

### Pharmaceutical compositions

In certain embodiments, the disclosure relates to pharmaceutical compositions comprising a compound disclosed herein and a pharmaceutically acceptable excipient. In certain embodiments, the pharmaceutical composition is in the form of a pill, capsule, tablet, or saline aqueous buffer.

In certain embodiments, the pharmaceutically acceptable excipient is selected from a saccharide, disaccharide, sucrose, lactose, glucose, mannitol, sorbitol, polysaccharides, starch, cellulose, microcrystalline cellulose, cellulose ether, hydroxypropyl cellulose (HPC), xylitol, sorbitol, maltito, gelatin, polyvinylpyrrolidone (PVP), polyethylene glycol (PEG), hydroxypropyl methylcellulose (HPMC), crosslinked sodium carboxymethyl cellulose, dibasic calcium phosphate, calcium carbonate, stearic acid, magnesium stearate, talc, magnesium carbonate, silica, vitamin A, vitamin E, vitamin C, retinyl palmitate, selenium, cysteine, methionine, citric acid, and sodium citrate, methyl paraben, propyl paraben, and combinations thereof.

Pharmaceutical compositions disclosed herein may be in the form of pharmaceutically acceptable salts, as generally described below. Some preferred, but non-limiting examples of suitable pharmaceutically acceptable organic and/or inorganic acids are hydrochloric acid, hydrobromic acid, sulfuric acid, nitric acid, acetic acid and citric acid, as well as other pharmaceutically acceptable acids known per se (for which reference is made to the references referred to below).

When the compounds of the disclosure contain an acidic group as well as a basic group, the compounds of the disclosure may also form internal salts, and such compounds are within the scope of the disclosure. When the compounds of the disclosure contain a hydrogen-donating heteroatom (e.g. NH), the disclosure covers salts and/or isomers formed by transfer of said hydrogen atom to a basic group or atom within the molecule.

Pharmaceutically acceptable salts of the compounds include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002).

Pharmaceutical compositions for use in the present disclosure typically comprise an effective amount of a compound and a suitable pharmaceutical acceptable carrier. The preparations may be prepared in a manner known per se, which usually involves mixing the at least one compound according to the disclosure with the one or more pharmaceutically acceptable carriers, and, if desired, in combination with other pharmaceutical active compounds, when necessary under aseptic conditions. Reference is made to U.S. Pat. No. 6,372,778, U.S. Pat. No. 6,369,086, U.S. Pat. No. 6,369,087 and U.S. Pat. No. 6,372,733 and the further references mentioned above, as well as to the standard handbooks, such as the latest edition of Remington's Pharmaceutical Sciences.

Generally, for pharmaceutical use, the compounds may be formulated as a pharmaceutical preparation comprising at least one compound and at least one pharmaceutically acceptable carrier, diluent or excipient and/or adjuvant, and optionally one or more further pharmaceutically active compounds.

The pharmaceutical preparations of the disclosure are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 1 and 1000 mg, and usually between 5 and 500 mg, of the at least one compound of the disclosure, e.g. about 10, 25, 50, 100, 200, 300 or 400 mg per unit dosage.

The compounds may be administered by a variety of routes including the oral, ocular, rectal, transdermal, subcutaneous, intravenous, intramuscular or intranasal routes, depending mainly on the specific preparation used. The compound will generally be administered in an "effective amount", by which is meant any amount of a compound that, upon suitable administration, is sufficient to achieve the desired therapeutic or prophylactic effect in the subject to which it is administered. Usually, depending on the condition to be prevented or treated and the route of administration, such an effective amount will usually be between 0.01 to 1000 mg per kilogram body weight of the patient per day, more often between 0.1 and 500 mg, such as between 1 and 250 mg, for example about 5, 10, 20, 50, 100, 150, 200 or 250 mg, per kilogram body weight of the patient per day, which may be administered as a single daily dose, divided over one or more daily doses. The amount(s) to be administered, the route of administration and the further treatment regimen may be determined by the treating clinician, depending on factors such as the age, gender and general condition of the patient and the nature and severity of the disease/symptoms to be treated. Reference is made to U.S. Pat. No. 6,372,778, U.S. Pat. No. 6,369,086, U.S. Pat. No. 6,369,087 and U.S. Pat. No. 6,372,733 and the further references mentioned above, as well as to the standard handbooks, such as the latest edition of Remington's Pharmaceutical Sciences.

For an oral administration form, the compound may be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation may be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, the compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the disclosure or their physiologically tolerable salts in a pharmaceutically acceptable solvent, such as ethanol or water, or a mixture of such solvents. If required, the formulation may contain other pharmaceutical auxiliaries such as surfactants, emulsifiers and stabilizers as well as a propellant.

For subcutaneous or intravenous administration, the compounds, if desired with the substances customary therefore such as solubilizers, emulsifiers or further auxiliaries are brought into solution, suspension, or emulsion. The compounds may also be lyophilized and the lyophilizates obtained used, for example, for the production of injection or infusion preparations. Suitable solvents are, for example, water, physiological saline solution or alcohols, e.g. ethanol, propanol, glycerol, sugar solutions such as glucose or mannitol solutions, or mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, the formulations may be prepared by mixing the compounds with a suitable non-irritating excipient, such as cocoa butter, synthetic glyceride esters or polyethylene glycols, which are solid at ordinary temperatures, but liquefy and/or dissolve in the rectal cavity to release the drug.

In certain embodiments, it is contemplated that these compositions may be extended release formulations. Typical extended release formations utilize an enteric coating. Typically, a barrier is applied to oral medication that controls the location in the digestive system where it is absorbed. Enteric coatings prevent release of medication before it reaches the small intestine. Enteric coatings may contain polymers of polysaccharides, such as maltodextrin, xanthan, scleroglucan dextran, starch, alginates, pullulan, hyaloronic acid, chitin, chitosan and the like; other natural polymers, such as proteins (albumin, gelatin etc.), poly-L-lysine; sodium poly(acrylic acid); poly(hydroxyalkylmethacrylates) (for example poly(hydroxyethyl methacrylate)); carboxypolymethylene (for example Carbopol^{™}); carbomer; polyvinylpyrrolidone; gums, such as guar gum, gum arabic, gum karaya, gum ghatti, locust bean gum, tamarind gum, gellan gum, gum tragacanth, agar, pectin, gluten and the like; poly(vinyl alcohol); ethylene vinyl alcohol; polyethylene glycol (PEG); and cellulose ethers, such as hydroxymethylcellulose (HMC), hydroxyethylcellulose (HEC), hydroxypropylcellulose (HPC), methylcellulose (MC), ethylcellulose (EC), carboxyethylcellulose (CEC), ethylhydroxyethylcellulose (EHEC), carboxymethylhydroxyethylcellulose (CMHEC), hydroxypropylmethyl-cellulose (HPMC), hydroxypropylethylcellulose (HPEC) and sodium carboxymethylcellulose (Na CMC); as well as copolymers and/or (simple) mixtures of any of the above polymers. Certain of the above-mentioned polymers may further be crosslinked by way of standard techniques.

The choice of polymer will be determined by the nature of the active ingredient/drug that is employed in the composition of the invention as well as the desired rate of release. In particular, it will be appreciated by the skilled person, for example in the case of HPMC, that a higher molecular weight will, in general, provide a slower rate of release of drug from the composition. Furthermore, in the case of HPMC, different degrees of substitution of methoxyl groups and hydroxypropoxyl groups will give rise to changes in the rate of release of drug from the composition. In this respect, and as stated above, it may be desirable to provide compositions of the invention in the form of coatings in which the polymer carrier is provided by way of a blend of two or more polymers of, for example, different molecular weights in order to produce a particular required or desired release profile.

Microspheres of polylactide, polyglycolide, and their copolymers poly(lactide-co-glycolide) may be used to form sustained-release protein or compound delivery systems. Proteins and/or compounds may be entrapped in the poly(lactide-co-glycolide) microsphere depot by a number of methods, including formation of a water-in-oil emulsion with water-borne protein and organic solvent-borne polymer (emulsion method), formation of a solid-in-oil suspension with solid protein dispersed in a solvent-based polymer solution (suspension method), or by dissolving the protein in a solvent-based polymer solution (dissolution method). One may attach poly(ethylene glycol) to proteins (PEGylation) to increase the in vivo half-life of circulating therapeutic proteins and decrease the chance of an immune response.

### Compound for use as a medicament

This disclosure relates to the asparagine endopeptidase inhibitors for use in the treatment or prevention of metastasis, tumor growth, and/or cancer. In certain embodiments, the disclosure relates to a pharmaceutical composition comprising a compound disclosed herein for use as a medicament for treating a cancer. In certain embodiments, the cancer is breast cancer, prostate cancer, colorectal cancer, gastric cancer, lung cancer, skin cancer, bladder cancer, brain cancer, kidney cancer, endometrial cancer, pancreatic cancer, and thyroid cancer.

"Cancer" refers any of various cellular diseases with malignant neoplasms characterized by the proliferation of cells. It is not intended that the diseased cells must actually invade surrounding tissue and metastasize to new body sites. Cancer can involve any tissue of the body and have many different forms in each body area. Within the context of certain embodiments, whether "cancer is reduced" may be identified by a variety of diagnostic manners known to one skill in the art including, but not limited to, observation the reduction in size or number of tumor masses or if an increase of apoptosis of cancer cells observed, e.g., if more than a 5 % increase in apoptosis of cancer cells is observed for a sample compound compared to a control without the compound. It may also be identified by a change in relevant biomarker or gene expression profile, such as PSA for prostate cancer, HER2 for breast cancer, or others.

The cancer to be treated in the context of the present disclosure may be any type of cancer or tumor. These tumors or cancer include, and are not limited to, tumors of the hematopoietic and lymphoid tissues or hematopoietic and lymphoid malignancies, tumors that affect the blood, bone marrow, lymph, and lymphatic system. Hematological malignancies may derive from either of the two major blood cell lineages: myeloid and lymphoid cell lines. The myeloid cell line normally produces granulocytes, erythrocytes, thrombocytes, macrophages and mast cells; the lymphoid cell line produces B, T, NK and plasma cells. Lymphomas, lymphocytic leukemias, and myeloma are from the lymphoid line, while acute and chronic myelogenous leukemia, myelodysplastic syndromes and myeloproliferative diseases are myeloid in origin.

Also contemplated are malignancies located in the colon, abdomen, bone, breast, digestive system, liver, pancreas, peritoneum, endocrine glands (adrenal, parathyroid, hypophysis, testicles, ovaries, thymus, thyroid), eye, head and neck, nervous system (central and peripheral), lymphatic system, pelvis, skin, soft tissue, spleen, thorax and genito-urinary apparatus and, more particularly, childhood acute lymphoblastic leukemia, acute lymphoblastic leukemia, acute lymphocytic leukemia, acute myeloid leukemia, adrenocortical carcinoma, adult (primary) hepatocellular cancer, adult (primary) liver cancer, adult acute lymphocytic leukemia, adult acute myeloid leukemia, adult Hodgkin's disease, adult Hodgkin's lymphoma, adult lymphocytic leukemia, adult non-Hodgkin's lymphoma, adult primary liver cancer, adult soft tissue sarcoma, AIDS-related lymphoma, AIDS-related malignant tumors, anal cancer, astrocytoma, cancer of the biliary tract, cancer of the bladder, bone cancer, brain stem glioma, brain tumors, breast cancer, cancer of the renal pelvis and ureter, primary central nervous system lymphoma, central nervous system lymphoma, cerebellar astrocytoma, brain astrocytoma, cancer of the cervix, childhood (primary) hepatocellular cancer, childhood (primary) liver cancer, childhood acute lymphoblastic leukemia, childhood acute myeloid leukemia, childhood brain stem glioma, childhood cerebellar astrocytoma, childhood brain astrocytoma, childhood extracranial germ cell tumors, childhood Hodgkin's disease, childhood Hodgkin's lymphoma, childhood visual pathway and hypothalamic glioma, childhood lymphoblastic leukemia, childhood medulloblastoma, childhood non-Hodgkin's lymphoma, childhood supratentorial primitive neuroectodermal and pineal tumors, childhood primary liver cancer, childhood rhabdomyosarcoma, childhood soft tissue sarcoma, childhood visual pathway and hypothalamic glioma, chronic lymphocytic leukemia, chronic myeloid leukemia, cancer of the colon, cutaneous T-cell lymphoma, endocrine pancreatic islet cells carcinoma, endometrial cancer, ependymoma, epithelial cancer, cancer of the oesophagus, Ewing's sarcoma and related tumors, cancer of the exocrine pancreas, extracranial germ cell tumor, extragonadal germ cell tumor, extrahepatic biliary tract cancer, cancer of the eye, breast cancer in women, Gaucher's disease, cancer of the gallbladder, gastric cancer, gastrointestinal carcinoid tumor, gastrointestinal tumors, germ cell tumors, gestational trophoblastic tumor, tricoleukemia, head and neck cancer, hepatocellular cancer, Hodgkin's disease, Hodgkin's lymphoma, hypergammaglobulinemia, hypopharyngeal cancer, intestinal cancers, intraocular melanoma, islet cell carcinoma, islet cell pancreatic cancer, Kaposi's sarcoma, cancer of kidney, cancer of the larynx, cancer of the lip and mouth, cancer of the liver, cancer of the lung, lymphoproliferative disorders, macroglobulinemia, breast cancer in men, malignant mesothelioma, malignant thymoma, medulloblastoma, melanoma, mesothelioma, occult primary metastatic squamous neck cancer, primary metastatic squamous neck cancer, metastatic squamous neck cancer, multiple myeloma, multiple myeloma/plasmatic cell neoplasia, myelodysplastic syndrome, myelogenous leukemia, myeloid leukemia, myeloproliferative disorders, paranasal sinus and nasal cavity cancer, nasopharyngeal cancer, neuroblastoma, non-Hodgkin's lymphoma during pregnancy, non-melanoma skin cancer, non-small cell lung cancer, metastatic squamous neck cancer with occult primary, buccopharyngeal cancer, malignant fibrous histiocytoma, malignant fibrous osteosarcoma/histiocytoma of the bone, epithelial ovarian cancer, ovarian germ cell tumor, ovarian low malignant potential tumor, pancreatic cancer, paraproteinemias, purpura, parathyroid cancer, cancer of the penis, phaeochromocytoma, hypophysis tumor, neoplasia of plasmatic cells/multiple myeloma, primary central nervous system lymphoma, primary liver cancer, prostate cancer, rectal cancer, renal cell cancer, cancer of the renal pelvis and ureter, retinoblastoma, rhabdomyosarcoma, cancer of the salivary glands, sarcoidosis, sarcomas, skin cancer, small cell lung cancer, small intestine cancer, soft tissue sarcoma, squamous neck cancer, stomach cancer, pineal and supratentorial primitive neuroectodermal tumors, T-cell lymphoma, testicular cancer, thymoma, thyroid cancer, transitional cell cancer of the renal pelvis and ureter, transitional renal pelvis and ureter cancer, trophoblastic tumors, cell cancer of the renal pelvis and ureter, cancer of the urethra, cancer of the uterus, uterine sarcoma, vaginal cancer, optic pathway and hypothalamic glioma, cancer of the vulva, Waldenstrom's macroglobulinemia, Wilms' tumor and any other hyperproliferative disease, as well as neoplasia, located in the system of a previously mentioned organ.

In certain embodiments compounds disclosed herein are combined with another anticancer agent. In certain embodiments, the anti-cancer agent selected from abemaciclib, abiraterone acetate, methotrexate, paclitaxel, adriamycin, acalabrutinib, brentuximab vedotin, ado-trastuzumab emtansine, aflibercept, afatinib, netupitant, palonosetron, imiquimod, aldesleukin, alectinib, alemtuzumab, pemetrexed disodium, copanlisib, melphalan, brigatinib, chlorambucil, amifostine, aminolevulinic acid, anastrozole, apalutamide, aprepitant, pamidronate disodium, exemestane, nelarabine, arsenic trioxide, ofatumumab, atezolizumab, bevacizumab, avelumab, axicabtagene ciloleucel, axitinib, azacitidine, carmustine, belinostat, bendamustine, inotuzumab ozogamicin, bevacizumab, bexarotene, bicalutamide, bleomycin, blinatumomab, bortezomib, bosutinib, brentuximab vedotin, brigatinib, busulfan, irinotecan, capecitabine, fluorouracil, carboplatin, carfilzomib, ceritinib, daunorubicin, cetuximab, cisplatin, cladribine, cyclophosphamide, clofarabine, cobimetinib, cabozantinib-S-malate, dactinomycin, crizotinib, ifosfamide, ramucirumab, cytarabine, dabrafenib, dacarbazine, decitabine, daratumumab, dasatinib, defibrotide, degarelix, denileukin diftitox, denosumab, dexamethasone, dexrazoxane, dinutuximab, docetaxel, doxorubicin, durvalumab, rasburicase, epirubicin, elotuzumab, oxaliplatin, eltrombopag olamine, enasidenib, enzalutamide, eribulin, vismodegib, erlotinib, etoposide, everolimus, raloxifene, toremifene, panobinostat, fulvestrant, letrozole, filgrastim, fludarabine, flutamide, pralatrexate, obinutuzumab, gefitinib, gemcitabine, gemtuzumab ozogamicin, glucarpidase, goserelin, propranolol, trastuzumab, topotecan, palbociclib, ibritumomab tiuxetan, ibrutinib, ponatinib, idarubicin, idelalisib, imatinib, talimogene laherparepvec, ipilimumab, romidepsin, ixabepilone, ixazomib, ruxolitinib, cabazitaxel, palifermin, pembrolizumab, ribociclib, tisagenlecleucel, lanreotide, lapatinib, olaratumab, lenalidomide, lenvatinib, leucovorin, leuprolide, lomustine, trifluridine, olaparib, vincristine, procarbazine, mechlorethamine, megestrol, trametinib, temozolomide, methylnaltrexone bromide, midostaurin, mitomycin C, mitoxantrone, plerixafor, vinorelbine, necitumumab, neratinib, sorafenib, nilutamide, nilotinib, niraparib, nivolumab, tamoxifen, romiplostim, sonidegib, omacetaxine, pegaspargase, ondansetron, osimertinib, panitumumab, pazopanib, interferon alfa-2b, pertuzumab, pomalidomide, mercaptopurine, regorafenib, rituximab, rolapitant, rucaparib, siltuximab, sunitinib, thioguanine, temsirolimus, thalidomide, thiotepa, trabectedin, valrubicin, vandetanib, vinblastine, vemurafenib, vorinostat, zoledronic acid, or combinations thereof such as cyclophosphamide, methotrexate, 5-fluorouracil (CMF); doxorubicin, cyclophosphamide (AC); mustine, vincristine, procarbazine, prednisolone (MOPP); sdriamycin, bleomycin, vinblastine, dacarbazine (ABVD); cyclophosphamide, doxorubicin, vincristine, prednisolone (CHOP); rituximab, cyclophosphamide, doxorubicin, vincristine, prednisolone (RCHOP); bleomycin, etoposide, cisplatin (BEP); epirubicin, cisplatin, 5-fluorouracil (ECF); epirubicin, cisplatin, capecitabine (ECX); methotrexate, vincristine, doxorubicin, cisplatin (MVAC).

In certain embodiments, the anti-cancer agent is an anti-PD-1, anti-CTLA4 antibody or combinations thereof, such as an anti-CTLA4 (e.g., ipilimumab, tremelimumab) and anti-PD1 (e.g., nivolumab, pembrolizumab, atezolizumab, avelumab, durvalumab). In certain embodiments, the method of administration is in a subject with a lymphodepleted environment. In certain embodiments, lymphodepleting agents (e.g., cyclophosphamide and fludarabine).

In certain embodiments, the disclosure contemplates the compounds disclosed herein and one more other anti-cancer agents for use as a medicament for treating breast cancer . In certain embodiments, the disclosure contemplates the compounds disclosed herein and trastuzumab and/or lapatinib for use as a medicament for treating breast cancer. In certain embodiments, the disclosure contemplates the compounds disclosed herein and docetaxel and cyclophosphamide for use as a medicament for treating breast cancer. In certain embodiments, the disclosure contemplates the compounds disclosed herein and docetaxel, carboplatin, and trastuzumab for use as a medicament for treating breast cancer . In certain embodiments, the disclosure contemplates the compounds disclosed herein and cyclophosphamide, doxorubicin, and 5-fluorouracil (5-FU) for use as a medicament for treating breast cancer . In certain embodiments, the disclosure contemplates the compounds disclosed herein and docetaxel, doxorubicin, and cyclophosphamide for use as a medicament for treating breast cancer . In certain embodiments, the disclosure contemplates the compounds disclosed herein and doxorubicin and cyclophosphamide followed by paclitaxel or docetaxel for use as a medicament for treating breast cancer . In certain embodiments, the disclosure contemplates the compounds disclosed herein and 5-FU, epirubicin, and cyclophosphamide followed by docetaxel or paclitaxel for use as a medicament for treating breast cancer .

In certain embodiments, the disclosure contemplates the compounds disclosed herein and one more other anti-cancer agents for use as a medicament for treating prostate cancer . In certain embodiments, the disclosure contemplates the compounds disclosed herein and leuprolide, goserelin, or buserelin for use as a medicament for treating prostate cancer . In certain embodiments, the disclosure contemplates the compounds disclosed herein and flutamide, bicalutamide, enzalutamide, or nilutamide for use as a medicament for treating prostate cancer . In certain embodiments, the disclosure contemplates the compounds disclosed herein and ketoconazole or aminoglutethimide for use as a medicament for treating prostate cancer . In certain embodiments, the disclosure contemplates the compounds disclosed herein and abiraterone, bicalutamide, cabazitaxel, bicalutamide, degarelix, denosumab, docetaxel, enzalutamide, cabazitaxel, leuprolide, prednisone, denosumab, sipuleucel-T, or radium 223 dichloride and combinations thereof for use as a medicament for treating prostate cancer .

In certain embodiments, the disclosure contemplates the compounds disclosed herein and one more other anti-cancer agents for use as a medicament for treating colon cancer . In certain embodiments, the disclosure contemplates the compounds disclosed herein and 5-FU, leucovorin, or capecitabine or combinations thereof for use as a medicament for treating colon cancer . In certain embodiments, the disclosure contemplates the compounds disclosed herein and capecitabine and oxaliplatin for use as a medicament for treating colon cancer . In certain embodiments, the disclosure contemplates the compounds disclosed herein and 5-FU, leucovorin, and oxaliplatin for use as a medicament for treating colon cancer . In certain embodiments, the disclosure contemplates the compounds disclosed herein and leucovorin, 5-FU, and irinotecan for use as a medicament for treating colon cancer . In certain embodiments, the disclosure contemplates the compounds disclosed herein and leucovorin, 5-FU, oxaliplatin, and irinotecan for use as a medicament for treating colon cancer .

In certain embodiments, the disclosure contemplates the compounds disclosed herein and bevacizumab or cetuximab for use as a medicament for treating colon cancer . In certain embodiments, the disclosure contemplates the compounds disclosed herein and 5-FU and leucovorin optionally with bevacizumab for use as a medicament for treating colon cancer . In certain embodiments, the disclosure contemplates the compounds disclosed herein and capecitabine optionally with bevacizumab for use as a medicament for treating colon cancer . In certain embodiments, the disclosure contemplates the compounds disclosed herein and irinotecan optionally with cetuximab for use as a medicament for treating colon cancer . In certain embodiments, the disclosure contemplates the compounds disclosed herein and cetuximab for use as a medicament for treating colon cancer . In certain embodiments, the disclosure contemplates the compounds disclosed herein and panitumumab for use as a medicament for treating colon cancer . In certain embodiments, the disclosure contemplates the compounds disclosed herein and regorafenib for use as a medicament for treating colon cancer .

In certain embodiments, the disclosure contemplates the compounds disclosed herein and a chemotherapy agent selected from vinorelbine, etoposide, mitomycin C, gemcitabine, irinotecan, pemetrexed, gefitinib, erlotinib, lapatinib, crizotinib, and a vinca alkaloid or combinations thereof for use as a medicament for treating lung cancer . In certain embodiments, the vinca alkaloid is vinblastine, vincristine, vindesine, or vinorelbine. In certain embodiments, the disclosure contemplates the compounds disclosed herein and chemotherapy agent is bevacizumab panitumumab, zalutumumab, nimotuzumab, matuzumab, or cetuximab for use as a medicament for treating lung cancer . In certain embodiments, the disclosure contemplates the compounds disclosed herein and a platinum-based agent and/or a taxane e.g., paclitaxel and docetaxel or combinations thereof for use as a medicament for treating lung cancer .

In certain embodiments, the disclosure contemplates the compounds disclosed herein, e.g., 7-morpholinobenzo[c][1,2,5]oxadiazol-4-amine or optionally substituted derivative or salt thereof to a subject in need thereof for use as a medicament for treating brain cancer, glioblastoma multiforme, oligodendroglioma, primitive neuroectodermal tumours, ependymomas, glioma comprising . In certain embodiments, the compound is optionally used in combination with temozolomide, procarbazine, carmustine (BCNU), lomustine (CCNU), vincristine, and combinations thereof. In certain embodiments, procarbazine, lomustine (CCNU) and vincristine are combined. In certain embodiments, the compound is optionally used in combination with irinotecan, cis-platin, carboplatin, methotrexate, etoposide, bleomycin, vinblastine, actinomycin (Dactinomycin), cyclophosphamide, or ifosfamide.

In certain embodiments, the disclosure contemplates combinations of compounds disclosed herein with temozolomide. Treatment of glioblastoma includes chemotherapy during and after radiotherapy.

In certain embodiments, the disclosure relates to administering compositions disclosed herein for the management of cancers or tumors in the brain by convection-enhanced delivery (CED). CED is a method of administrating compositions by direct infusion into the brain interstitial spaces utilizing a fluid pressure gradient after catheter placement.

The cancer treatments disclosed herein can be applied as a sole therapy or can involve, conventional surgery or radiotherapy or chemotherapy. Such chemotherapy can include one or more of the following categories of anti-tumor agents:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulfan and nitrosoureas); antimetabolites (for example antifolates such as fluoropyrimidines like 5-fluorouracil and gemcitabine, tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin); and proteosome inhibitors (for example bortezomib [Velcade^{®}]); and the agent anegrilide [Agrylin^{®}]; and the agent alpha- interferon
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-Her2 antibody trastuzumab and the anti- epidermal growth factor receptor (EGFR) antibody, cetuximab) , farnesyl transferase inhibitors, tyrosine kinase inhibitors and serine/threonine kinase inhibitors, for example inhibitors of the epidermal growth factor family for example EGFR family tyrosine kinase inhibitors such as: N-(3-chloro-4-fluorophenyl)-7-methoxy-6-(3-morpholinopropoxy)quinazolin-4-a mine (gefitinib), N-(3-ethynylphenyl)-6,7-bis(2-methoxyethoxy)quinazolin-4-amine (erlotinib), and 6-acrylamido-N-(3-chloro-4-fluorophenyl)-7-(3-morpholinopropoxy)quinazolin-4-amine (CI 1033), for example inhibitors of the platelet-derived growth factor family and for example inhibitors of the hepatocyte growth factor family, for example inhibitors of phosphotidylinositol 3-kinase (PI3K) and for example inhibitors of mitogen activated protein kinase kinase (MEK1/2) and for example inhibitors of protein kinase B (PKB/Akt), for example inhibitors of Src tyrosine kinase family and/or Abelson (AbI) tyrosine kinase family such as dasatinib (BMS-354825) and imatinib mesylate (Gleevec^{™}); and any agents that modify STAT signalling;
(v) antiangiogenic agents such as those which inhibit the effects of vascular endothelial growth factor, (for example the anti-vascular endothelial cell growth factor antibody bevacizumab [Avastin^{™}]) and compounds that work by other mechanisms (for example linomide, inhibitors of integrin ocvβ3 function and angiostatin);
(vi) vascular damaging agents such as Combretastatin A4;
(vii) antisense therapies, for example those which are directed to the targets listed above, such as an anti-RAS antisense; and
(viii) immunotherapy approaches, including for example ex-vivo and in-vivo approaches to increase the immunogenicity of subject tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies, and approaches using the immunomodulatory drugs thalidomide and lenalidomide [Revlimid^{®}].

The combination therapy also contemplates use of the disclosed pharmaceutical compositions with radiation therapy or surgery, as an alternative, or a supplement, to a second therapeutic or chemotherapeutic agent.

In certain embodiments, the disclosure contemplates compounds disclosed herein for use in treating or preventing leukemia using a leukemia (CLL) chemotherapeutic plan. A typical chronic lymphocytic leukemia (CLL) chemotherapeutic plan includes combination chemotherapy with chlorambucil or cyclophosphamide, plus a corticosteroid such as prednisone or prednisolone. The use of a corticosteroid has the additional benefit of suppressing some related autoimmune diseases, such as immunohemolytic anemia or immune-mediated thrombocytopenia. In resistant cases, single-agent treatments with nucleoside drugs such as fludarabine, pentostatin, or cladribine may be successful. Patients may consider allogeneic or autologous bone marrow transplantation. In certain embodiments, the disclosure contemplates combination treatments using compounds disclosed herein in combination with chloroambucil, cyclophosphamide, prednisone, prednisolone, fludarabine, pentostatin, and/or cladribine or combinations thereof. Treatment of acute lymphoblastic leukemia typically includes chemotherapy to bring about bone marrow remission. Typical regiments include prednisone, vincristine, and an anthracycline drug, L-asparaginase or cyclophosphamide. Other options include prednisone, L-asparaginase, and vincristine. Consolidation therapy or intensification therapy to eliminate any remaining leukemia may include antimetabolite drugs such as methotrexate and 6-mercaptopurine (6-MP).

In certain embodiments, the disclosure contemplates combination treatments using compounds disclosed herein in combination with COP, CHOP, R-CHOP, imatinib, alemtuzumab, vincristine, L-asparaginase or cyclophosphamide, methotrexate and/or 6-mercaptopurine (6-MP). COP refers to a chemotherapy regimen used in the treatment of lymphoma of cyclophosphamide, vincristine, and prednisone or prednisolone and optionally hydroxydaunorubicin (CHOP) and optionally rituximab (R-CHOP).

In certain embodiments, the asparagine endopeptidase inhibitors are useful for treating or preventing neurodegenerative diseases and cognitive disorders such as Alzheimer's Disease. In certain embodiments, the disclosure relates to pharmaceutical compositions comprising an asparagine endopeptidase inhibitor and a pharmaceutically acceptable excipient. In certain embodiments, the disclosure relates to the compound according to the present invention for use in treating or preventing a neurodegenerative disease comprising administering an effective amount of pharmaceutical composition a asparagine endopeptidase inhibitor disclosed herein to a subject in need thereof.

In certain embodiments, the subject is at risk of or exhibiting symptoms of AD.

In certain embodiments, the disclosure contemplates administering compounds disclosed herein in combination with an imaging agent such as florbetapir (¹⁸F) and/or a therapeutic agent related to treating or ameliorating one or more symptoms of AD.

In certain embodiments, the disclosure contemplates administering compounds disclosed herein in combination with medications for memory loss, treatments for behavioral changes, treatments for sleep changes.

In certain embodiments, the disclosure contemplates administering compounds disclosed herein in combination with medication selected from cholinesterase inhibitors such as donepezil, rivastigmine, galantamine, and tacrine and/or an agent for blocking NMDA receptor such as memantine to treat the cognitive symptoms (memory loss, confusion, and problems with thinking and reasoning) of Alzheimer's disease.

In certain embodiments, the disclosure contemplates administering compounds disclosed herein in combination with Vitamin E.

In certain embodiments, the disclosure contemplates administering compounds disclosed herein in combination with medications such as anti-irritability, anti-anxiety, anti-psychotic, anti-insomnia, and anti-depression agents.

In certain embodiments, the disclosure contemplates administering compounds disclosed herein in combination with monoclonal antibody vaccines to amyloid including but not limited to solanuzemab, gantenerumab, and bapineuzumab.

In certain embodiments, the disclosure contemplates administering compounds disclosed herein in combination with medications for stroke or traumatic brain injury.

In certain embodiments, the disclosure contemplates administering compounds disclosed herein in combination with with recombinant tissue plasminogen activator (rtPA).

In certain embodiments, compounds disclosed herien can be used to treat a variety of diseases associated with apoptosis including neurodegenerative disorders, ischemic injuries, acquired immunodeficiency syndrome (AIDS), and osteoporosis. Apoptosis is involved in amyotrophic lateral sclerosis (ALS), Huntington's disease, Alzheimer's disease, Parkinson's disease, and spinal muscular atrophy. In multiple sclerosis (MS), the death of the oligodendrocytes is an important example of the glial degeneration through apoptosis.

In certain embodiments, compounds disclosed herein would be useful for the treatment of Huntington's disease and other neurodegenerative diseases such as dentatorubropallidoluysian atrophy (DRPLA), spinocerebellar atrophy type 3 (SCA-3), and spinal bulbar muscular atrophy (SBMA).

Neuronal apoptosis is also seen after acute injuries such as stroke, trauma, and ischemia. Apoptosis has been observed in striatal and cortical neurons in animal models of stroke.

### EXPERIMENTAL

Various concentrations of the compounds were incubated with AEP reaction buffer (50 mM Sodium Citrate pH 5.5, 0.1% CHAPS, 60 mM Na₂HPO₄, 1 mM EDTA, final pH 6.0) and peptide substrate, 10 µM Cbz-AAN-AMC. The reaction was initiated upon addition of 50 nM AEP and fluorescent product formation was monitored over 15 min. The IC₅₀ values were calculated from the following equation: Fractional Enzymatic Activity = 1 / (1 + ([I]/IC₅₀)), in which [I] = Inhibitor concentration and IC₅₀ = inhibitor concentration that yields half-maximal activity. Data were analyzed with GraFit version 5.0.11 software package.

### (7-((5-(methylamino)-1,3,4-thiadiazol-2-yl)amino)-4-morpholinobenzo[c][1,2,5]oxadiazol-5-yl)methanol

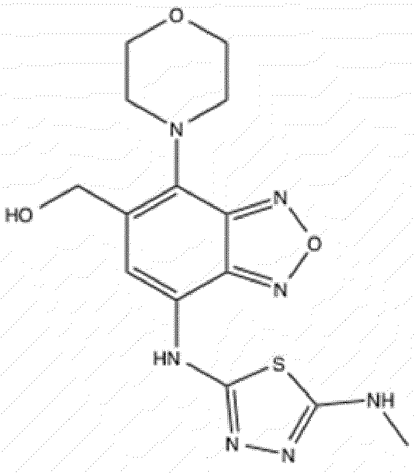

| In vitro Biology | Units | Value & Class |
|---|---|---|
| **Activity** | | |
| (assay 1)-EC₅₀ | nM | 10 nM |
| | | recombinant AEP proteins |

| **Selectivity** | | |
|---|---|---|
| (Assay 1 -IC₅₀/Fold selectivity) | nM | 3023 fold |
| | | 30.23 (µM) |
| | | AEP versus caspase-3 |

Compounds disclosed herein may be prepared using procedures outlined herein by substituting appropriate starting materials.

### Synthesis of (7-((5-(methylamino)-1,3,4-thiadiazol-2-yl)amino)-4-morpholinobenzo[c][1,2,5] oxadiazol-5-yl)methanol

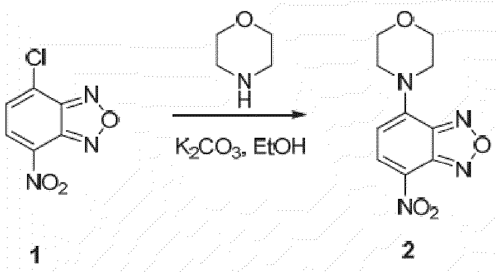

A mixture of compound 1 (5.00 g, 25.0 mmol) and K₂CO₃ (690 mg, 5.0 mmol) in ethanol (50 mL) was stirred at room temperature for 10 minutes, then morpholine (3.3 g, 37.6 mmol) was added to the reaction mixture, which was stirred at room temperature overnight. The reaction mixture was filtered and the solid cake was washed with water (20 mL) and ethanol (40 mL) to give compound 2 (6.15 g, 98.0 % yield) as a red solid. MS (ESI) *m*/*z* 251.1 [M+H]⁺

### Synthesis of 5-bromo-4-morpholino-7-nitrobenzo[c][1,2,5]oxadiazole (3)

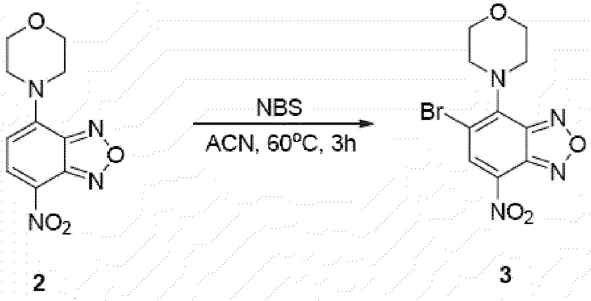

To a mixture of compound **2** (6.15 g, 24.6 mmol) in acetonitrile (100 mL) was added NBS (5.25 g, 29.5 mmol) at 0 °C. The resulting mixture was stirred at 60 °C for 3h. After the reaction was completed, the mixture was filtered, and the filtrate was concentrated under reduced pressure to give compound 3 as a red solid (7.30 g, 90% yield); MS (ESI) *m*/*z* 328.9, 330.9 [M+H]⁺.

### Synthesis of 6-bromo-7-morpholinobenzo[c][1,2,5]oxadiazol-4-amine (4)

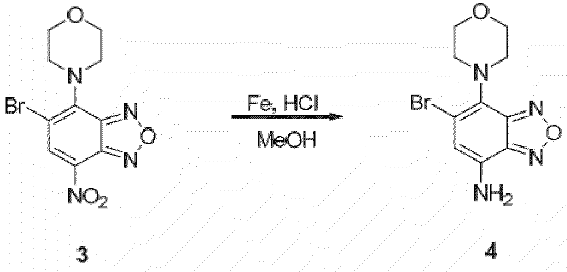

To a solution of compound **3** (1.00 g, 3.00 mmol) and and Fe (1.70 g, 30.4 mmol) in DCM (10 mL) / methanol (5 mL) was added conc. HCl (2 mL). The reaction mixture was stirred at room temperature overnight. The reaction mixture was filtered by Celite and the filtrate concentrated to get crude compound **4** (820 mg, 90 % yield) as a red solid; MS (ESI) *m*/*z* 299.0, 301.0 [M+H]⁺.

### Synthesis of tert-butyl(6-bromo-7-morpholinobenzo[c][1,2,5]oxadiazol-4-yl)carbamate (5)

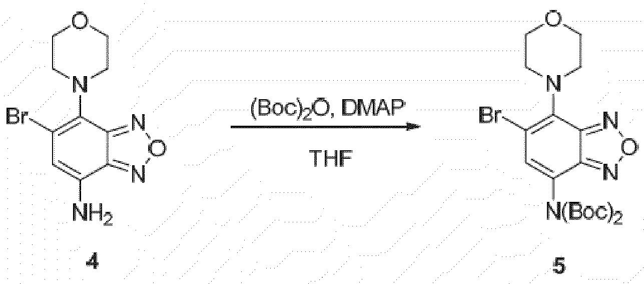

To a solution of compound **4** (900 mg, 3.0 mmol) in THF (10 mL) was added (Boc)₂O (1.38 g, 6.3 mmol) and DMAP (34 mg, 0.3 mmol). The reaction mixture was stirred at room temperature overnight. The reaction mixture was concentrated in vacuo to afford the residue, which was purified by column chromatography on silica gel (petroleum ether/EtOAc = 10:1) to give compound **5** (1.1 g, 73% yiled) as a red solid; MS (ESI) *m*/*z* 343.0 [M-156+H]⁺.

### Methyl 7-((tert-butoxycarbonyl)amino)-4-morpholinobenzo[c][1,2,5]oxadiazole-5-carboxylate (6)

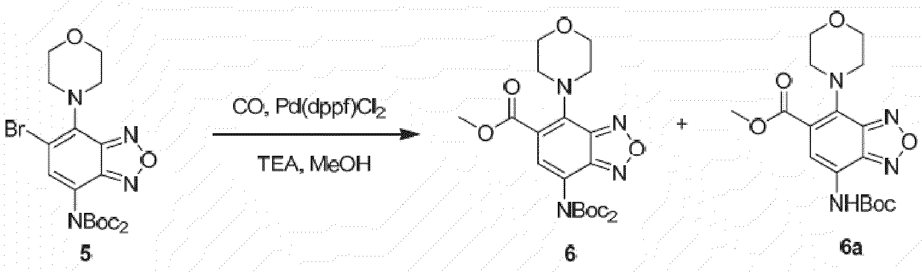

To a solution of compound **5** (3.00 g, 6.01 mmol) in MeOH (60 mL) was added Pd(dppf)Cl₂ (439 mg, 0.60 mmol) and TEA (1.50 g, 15.9 mmol). The reaction mixture was stirred at 85 °C under CO (1MPa) for 16 h. After cooling down to room temperature, the solvent was removed. The residue was purified by column chromatography on silica gel column (petroleum ether/EtOAc = 5:1) to afford a mixture of compound **6** and **6a** (2.30 g, crude) as a red solid. MS (ESI) *m*/*z* 479.2 and 379.1 [M+H]⁺.

### Methyl 7-amino-4-morpholinobenzo[c][1,2,5]oxadiazole-5-carboxylate (7)

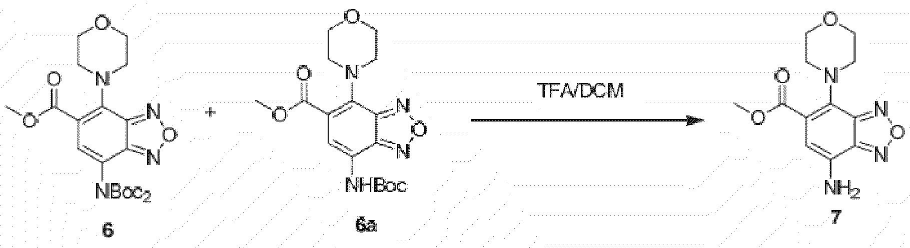

A mixture of compound **6** and **6a** (2.30 g, crude) in DCM (10 mL) at 0 °C was added TFA (5 mL) slowly. The reaction mixture was stirred at room temperature for 2 hours. After the solvent was removed, the residue was purified by Prep-HPLC (10-95% CH₃CN in H₂O, 0.5% ammonia) to afford the target compound (1.14 g, 68% yield, over 2 steps) as a red solid. MS (ESI) m/z 279.1 [M+H]⁺.

### Methyl 7-((5-((tert-butoxycarbonyl)(methyl)amino)-1,3,4-thiadiazol-2-yl)amino)-4-morpholinobenzo[c][1,2,5]oxadiazole-5-carboxylate (8)

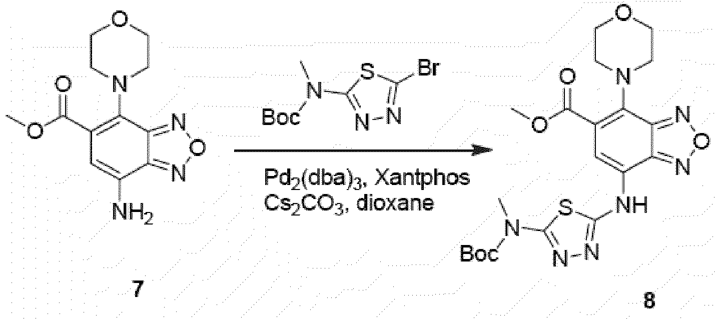

To a solution of compound **7** (1.14 g, 4.10 mmol) and tert-butyl (5-bromo-1,3,4-thiadiazol-2-yl)(methyl)carbamate (2.40 g, 8.16 mmol) in dioxane (10 mL) was added Pd₂(dba)₃ (376 mg, 0.40 mmol), Xantphos (237 mg, 0.40 mmol) and Cs₂CO₃ (2.70 g, 8.28 mmol). The reaction mixture was stirred at 100°C under N₂ for 3 hours. The solvent was removed, and the residue was purified by column chromatography on silica gel column (petroleum ether/EtOAc = 5:1) to afford compound **8** (1.12 g, crude) as a red solid. MS (ESI) *m*/*z* 492.2 [M+H]⁺.

### Tert-butyl (5-((6-(hydroxymethyl)-7-morpholinobenzo[c][1,2,5]oxadiazol-4-yl)amino)-1,3,4-thiadiazol-2-yl)(methyl)carbamate (9)

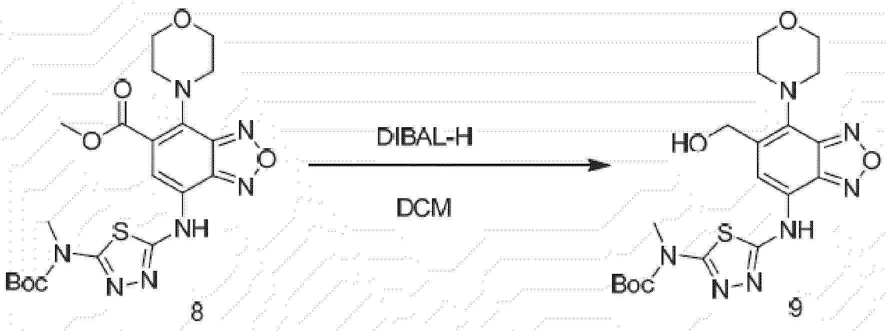

To a solution of compound **8** (1.12 g, crude) in DCM (50 mL) was added DIBAL-H (5.4 mL, 8.12 mmol, 1.5 M in toluene) dropwise at -78°C. The result mixture was stirred at -78°C for 1 hour. The reaction was quenched with NH₄Cl (aq), then the solvent was removed, and the residue was purified by column chromatography on silica gel column (petroleum ether/EtOAc = 3:1) to afford compound **9** (550 mg, crude) as a red solid. MS (ESI) *m*/*z* 464.2 [M+H]⁺.

### (7-((5-(Methylamino)-1,3,4-thiadiazol-2-yl)amino)-4-morpholinobenzo[c][1,2,5]oxadiazol-5-yl)methanol

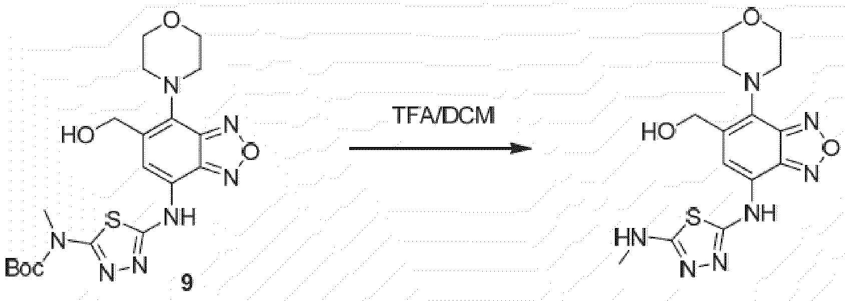

To a solution of compound **9** (550 mg, crude) in DCM (3 mL) was added TFA (1.5 mL) slowly at 0 °C. The reaction mixture was stirred at room temperature for 2 hours. After the solvent was removed, the residue was purified by prep-HPLC (10-70% CH₃CN in H₂O, 0.5% ammonia) to afford the target compound (125 mg, 8% yield, over 3 steps) as a yellow solid.¹H NMR (DMSO-d₆, 400MHz): δ (ppm) 9.67 (brs, 1H), 8.10 (s, 1H), 5.47 (brs, 1H), 4.69 (s, 2H), 3.77 - 3.75 (m, 4H), 3.69 (s, 3H), 3.15 - 3.13 (m, 4H). MS (ESI) m/z 364.1 [M+H]⁺.

### Tert-butyl (6-cyano-7-morpholinobenzo[c][1,2,5]oxadiazol-4-yl)carbamate (10)

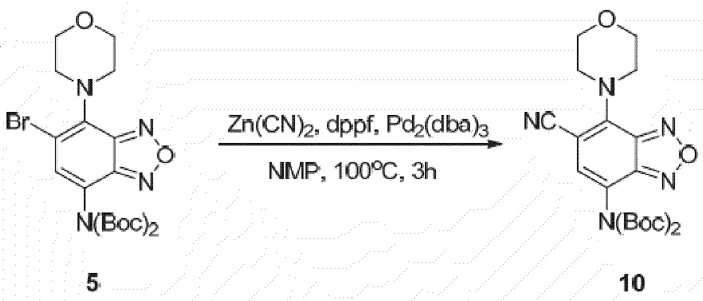

To a solution of compound **5** (3.00 g, 6.01 mmol) in NMP (10 mL) was added Zn(CN)₂ (1.40 g, 12.0 mmol), dppf (665 mg, 1.20 mmol), and Pd₂(dba)₃ (550 mg, 0.60 mmol). The reaction mixture was stirred at 100 °C for 3 h under N₂ protected. After cooling down to room temperature, then EtOAc and water was added. The separated organic layer was concentrated and the result residue was purified by chromatography on silica gel column (petroleum ether/EtOAc = 3:1) to afford compound **6** (1.93 g, 72% yield) as a red solid. MS (M-100+H⁺) m/z 346.1.

### 7-Amino-4-morpholinobenzo[c][1,2,5]oxadiazole-5-carbonitrile (11)

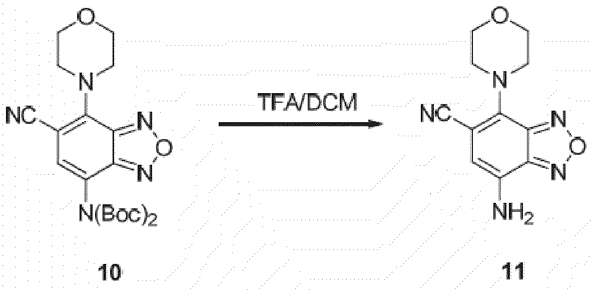

To a solution of compound **10** (1.93 g, 4.33 mmol) in DCM (4 mL) was added TFA (2 mL) slowly at 0 °C. The reaction mixture was stirred at room temperature for 2 hours. After the solvent was removed, the residue was purified by prep-HPLC (10-70% CH₃CN in H₂O, 0.5% ammonia) to afford the target compound (980 mg, 92% yield) as a red solid. MS (ESI) m/z 246.1 [M+H]⁺

### 7-((5-(Methylamino)-1,3,4-thiadiazol-2-yl)amino)-4-morpholinobenzo[c][1,2,5]oxadiazole-5-carbonitrile

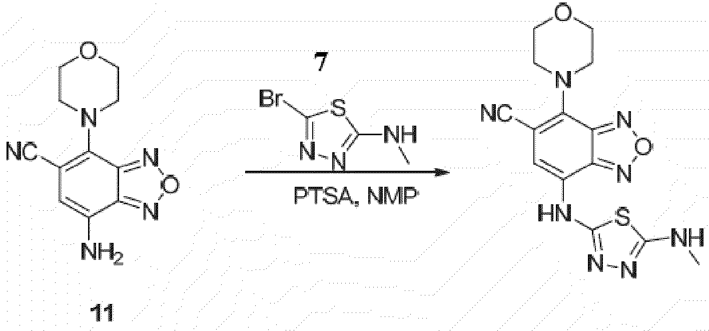

A mixture of compound 7 (500 mg, 2.0 mmol) and 5-bromo-N-methyl-1,3,4-thiadiazol-2-amine 11 (786 mg, 2.0 mmol), PTSA (422 mg, 2.5 mmol) in NMP (5 mL) was stirred at 150 °C under microwave irradiation for 8 hours. The reaction mixture was purified directly by prep-HPLC(10-95 CH₃CN in water) give the desired product (106 mg, 14% yield) as a red solid.¹H NMR (DMSO-*d₆*, 400MHz): δ (ppm) 10.56 (brs, 1H), 8.07 (s, 1H), 7.20 - 7.17 (m, 1H), 3.80-3.72 (s, 8H), 2.83 (d, *J=* 4.4 Hz, 3H). MS (ESI) m/z 359.1 [M+H]⁺.

### Synthesis of N²-methyl-N⁵-(7-morpholinobenzo[c][1,2,5]oxadiazol-4-yl)-1,3,4-thiadiazole-2,5-diamine

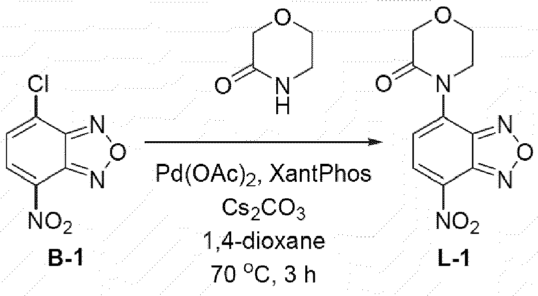

To a mixture of compound **B-1** (3 g, 15 mmol) and morpholin-3-one (1.8 g, 18 mmol) in dry 1,4-dioxane (90 mL) under Ar at room temperature were added Cs₂CO₃ (9.72 g,30 mmol), XantPhos (868 mg,1.5 mmol) and Pd(OAc)₂ (337 mg,1.5 mmol). The reaction was stirred under Ar at 70 °C for 3 h. LC-MS indicated part of **B-1** remained and the desired product was formed as major product. The cooled reaction mixture was filtered, and the filter cake was washed with DCM. The combined filtrate was concentrated in vacuo. The residue was purified by silica gel chromatography (eluent: PE/EtOAc from 6:1 to 1:1) to get desired product as a dark solid (1.3 g, yield: 20%, confirmed by ¹H NMR). ¹H NMR (400 MHz, CDCl₃) δ 8.55 (d, *J=* 8.0 Hz, 1H), 7.88 (d, *J=* 8.0 Hz, 1H), 4.49 (s, 2H), 4.28 (t, *J =* 5.0 Hz, 2H), 4.17 (t, *J =* 4.8 Hz, 2H).

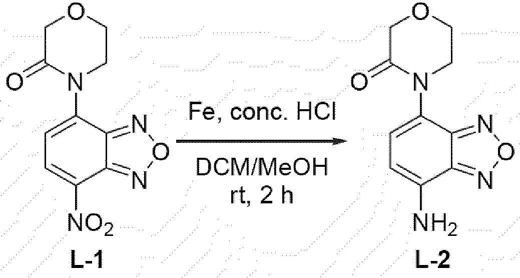

To a stirred mixture of compound **L-1** (1.3 g, 4.9 mmol) in DCM/MeOH (20 mL/4 mL) were added Fe (1.37 g,24.6 mmol) and conc. HCl (3 mL). The reaction mixture was stirred at room temperature for 2 h. TLC (PE:EA = 1:1) indicated the reaction was complete. The reaction mixture was basified with aqueous Na₂CO₃ till pH equals 8. The resultant was extracted with DCM. The combined organic extracts were concentrated in vacuo. The residue was purified by silica gel chromatography (eluent: PE:EtOAc from 2:1 to 1:3) to get desired **L-2** as a red solid (1 g, yield: 88%, confirmed by ¹H NMR and LC-MS). LC-MS purity: 83% @ 254 nm; [M+H]⁺ = 235.1, R.T. = 0.90 min. ¹H NMR (400 MHz, DMSO-*d₆*) δ 7.28 (d, *J =* 7.6 Hz, 1H), 6.72 (s, 2H), 6.29 (d, *J =* 7.6 Hz, 1H), 4.24 (s, 2H), 4.00 (t, *J=* 5.0 Hz, 4H), 3.73 (t, *J=* 4.8 Hz, 4H).

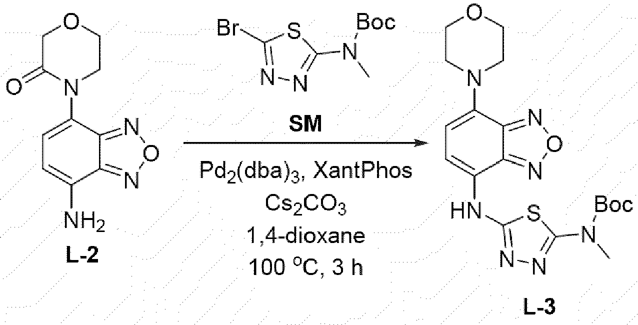

To a mixture of compound **L-2** (1.0 g, 4.27 mmol) and *tert*-butyl (5-bromo-1,3,4-thiadiazol-2-yl)(methyl)carbamate (1.88 g, 6.4 mmol) in dry 1,4-dioxane (100 mL) under Ar at room temperature were added Cs₂CO₃ (2.77 g, 8.45 mmol), XantPhos (247 mg, 0.47 mmol) and Pd₂(dba)₃ (391 mg, 0.427 mmol). The reaction was stirred under Ar at 100 °C for 3 h.

TLC (PE:EA = 1:1) indicated the reaction was complete. The reaction mixture was cooled to room temperature, filtered and the filter cake was washed with DCM. The combined filtrate was concentrated in vacuo. The residue was purified by silica gel chromatography (eluent: PE:EtOAc from 5:1 to 1:1) to get impure desired product as a red solid (600 mg, yield: ca. 31%).

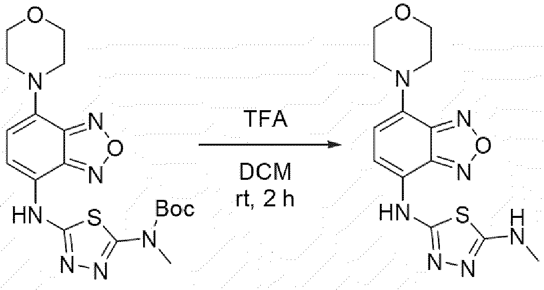

To a solution of **L-3** (600 mg, 1.34 mmol) in DCM (40 mL) was added TFA (3.04 g, 13.4 mmol). The reaction mixture was stirred at room temperature for 2 h. TLC (PE:EA = 1:2) indicated the reaction was complete. The reaction mixture was washed with aqueous NaHCO₃ and evaporated in vacuo. The residue was purified by prep-HPLC to get desired product as a red solid (90 mg, yield: 20%, confirmed by ¹H NMR, HPLC and LC-MS). HPLC purity: 99% @ 254 nm; 97% @ 214 nm. LC-MS: [M+H]⁺ = 348.2, R.T. = 0.90 min. ¹H NMR (400 MHz, DMSO-*d₆*) δ 10.84 (s, 1H), 8.05 (s, 1H), 7.53 (d, *J=* 7.2 Hz, 1H), 7.19 (s, 1H), 4.03 (t, *J =* 4.8 Hz, 4H), 3.81 (t, *J=* 4.6 Hz, 4H), 2.84 (t, *J =* 4.8 Hz, 3H).

### Synthesis of N²-(5-chloro-7-morpholinobenzo[c][1,2,5]oxadiazol-4-yl)-N⁵-methyl-1,3,4-thiadiazole-2,5-diamine

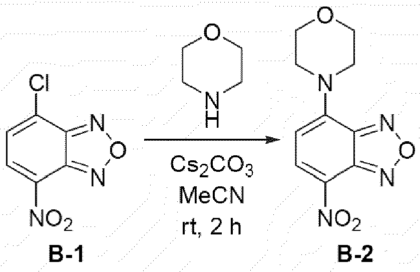

A solution of compound **B-1** (3.5 g, 17.5 mmol), morpholine (3.0 g, 34.5 mmol) and Cs₂CO₃ (17.0 g, 52.1 mmol) in MeCN (15 mL) was stirred at room temperature for 2 h. TLC indicated the reaction was complete. To the reaction mixture was added EtOAc (500 mL) and water (200 mL), red solid precipitated. The mixture was filtered. The filter cake was collected and dried to give 1^{st} portion of desired product. The organic layer of the filtrate was separated, washed with brine, dried over anhydrous Na₂SO₄, filtered and concentrated to give 2^{nd} portion of desired product (totally 4.4 g, yield: ca. 100%, , confirmed by ¹H NMR). ¹H NMR (400 MHz, CDCl₃) δ 8.46 (d, *J* = 8.8 Hz, 1H), 6.34 (d, *J=* 8.8 Hz, 1H), 4.09 (t, *J =* 4.8 Hz, 2H), 3.97 (t, *J =* 4.8 Hz, 2H).

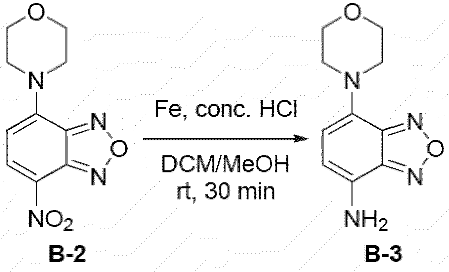

To a stirred mixture of compound **B-2** (4.7 g, 18.8 mmol) in DCM (370 mL) and MeOH (180 mL) at room temperature were added conc. HCl (36.5 wt%, 19 mL) and Fe (8.0 g, 142 mmol). The resulting mixture was stirred at room temperature for 30 min. To the reaction mixture was added saturated aqueous NaHCO₃ till pH equals 8. The organic layer was separated, washed with brine, dried over anhydrous Na₂SO₄, filtered and concentrated to give desired product as a red solid (4.0 g, yield: 97%, confirmed by ¹H NMR and LC-MS). LC-MS: [M+H]⁺ = 221.4, R.T. = 1.60 min. ¹H NMR (400 MHz, DMSO-*d₆*) δ 6.49 (d, *J =* 8.0 Hz, 1H), 6.26 (d, *J =* 7.6 Hz, 1H), 5.76 (brs, 2H), 3.78 (t, *J =* 4.6 Hz, 4H), 3.17 (t, *J =* 4.4 Hz, 4H).

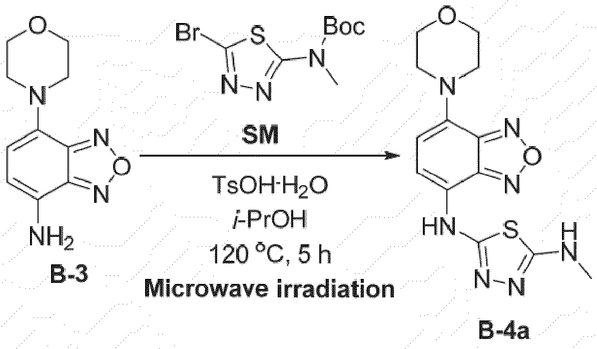

A mixture of **B-3** (1.0 g, 4.5 mmol), *tert*-butyl (5-bromo-1,3,4-thiadiazol-2-yl)(methyl)carbamate (1.5 g, 5.1 mmol) and TsOH·H₂O (960 mg, 5.0 mmol) in i-PrOH (10 mL) was stirred at 120 °C for 5 h with microwave irradiation. To the cooled reaction mixture was added saturated aq. NaHCO₃ till pH equals 8. DCM (200 mL) was added and the mixture was stirred for 10 min. The organic layer was separated, washed with brine, dried over anhydrous Na₂SO₄, filtered and concentrated. The crude product was purified by reversed phase chromatography to give impure desired product as a brown solid (650 mg, yield: 43%, confirmed by LC-MS). LC-MS purity: 68% @ 254 nm. LC-MS: [M+H]⁺ = 334.1, R.T. = 1.34 min.

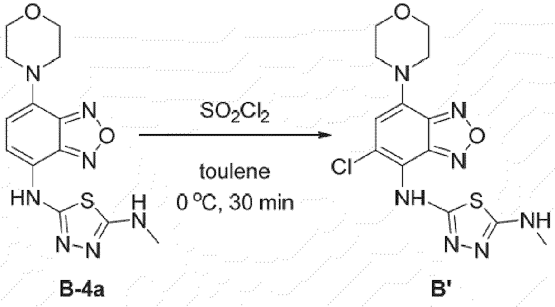

To a stirred solution of compound **B-4a** (400 mg, 1.2 mmol) in toluene (10 mL) at 0 °C was added SO₂Cl₂ (1.6 g, 12 mmol). The reaction was stirred at 0 °C for 30 min. LC-MS indicated the reaction was complete and the desired product was formed along with large amount of byproducts. To the reaction mixture was added PE and the precipitated yellow solid was collected by filtration. The yellow solid (low purity) was purified by prep-HPLC to give desired product as a brown solid (9.4 mg in vial, yield: 2.1%, confirmed by ¹H NMR, HPLC and LC-MS). HPLC purity: 91% @ 254 nm; 91% @ 214 nm. LC-MS: [M+H]⁺ = 368.0, R.T. = 5.14 min. 1H NMR (400 MHz, DMSO-*d₆*) δ 8.14 (s, 1H), 7.21 (d, *J=* 4.4 Hz, 1H), 3.76 (t, *J=* 4.4 Hz, 4H), 3.24 (t, *J* = 4.6 Hz, 4H), 2.84 (d, J = 4.8 Hz, 3H).

### N2-methyl-N5-(5-methyl-7-morpholinobenzo[c][1,2,5]oxadiazol-4-yl)-1,3,4-thiadiazole-2,5-diamine

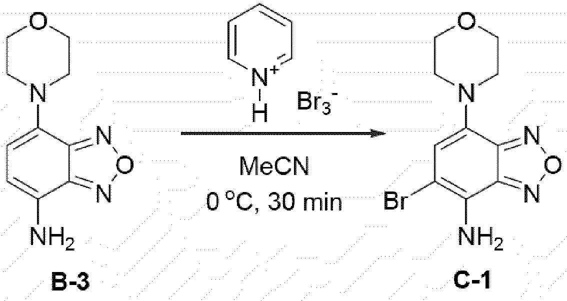

To a stirred solution of compound **B-3** (500 mg, 2.3 mmol) in MeCN (10 mL) at 0 °C was added pyridinium tribromide (950 mg, 2.95 mmol). The reaction was stirred at 0 °C for 30 min. LC-MS indicated the reaction was complete. To the reaction mixture was added water (100 mL) and EtOAc (300 mL). The organic layer was separated, washed with brine, dried over anhydrous Na₂SO₄, filtered and the filtrate was concentrated to give crude desired product, which was purified by reversed phase chromatography to give desired product as a red solid (400 mg, yield: 58%, confirmed by ¹H NMR and LC-MS).

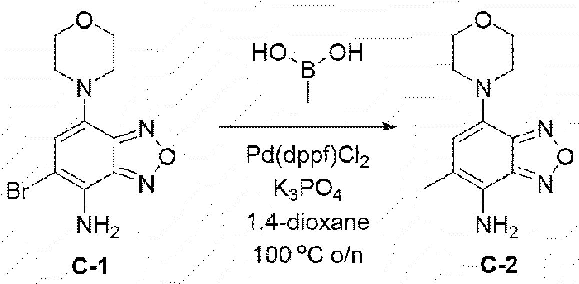

A mixture of compound **C-1** (370 mg, 1.2 mmol), methylboronic acid (450 mg, 7.4 mmol), K₂CO₃ (510 mg, 3.7 mmol) and Pd (PPh₃)₄ (140 mg, 0.12 mmol) in DMF (12 mL) was stirred at under argon at 120 °C for overnight. The cooled reaction mixture was directly purified by reversed phase chromatography to give desired product (150 mg, yield: 53%, confirmed by ¹H NMR) (Note: position of the methyl group was determined by 2D NMR). 1H NMR (400 MHz, CDCl₃) δ 6.25 (s, 1H), 4.10 (brs, 2H), 3.93 (t, *J =* 4.6 Hz, 4H), 3.33 (s, 4H), 2.18 (s, 3H).

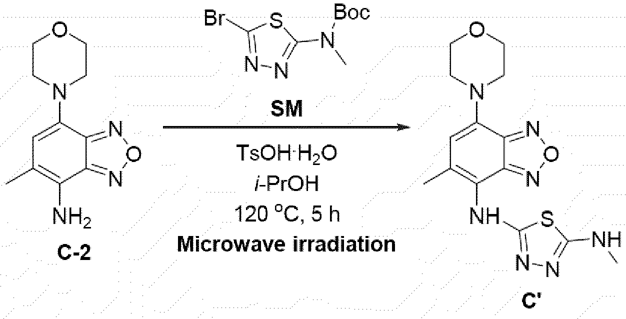

A solution of compound **C-2** (130 mg, 0.56 mmol), *tert*-butyl (5-bromo-1,3,4-thiadiazol-2-yl)(methyl)carbamate (250 mg, 1.83 mmol) and TsOH·H₂O (160 mg, 0.83 mmol) was stirred at 120 °C for 5 h with microwave irradiation. TLC indicated large amount of starting material remained. The cooled reaction mixture was purified by prep-TLC to afford desired product (20 mg, purity: 80%), which was further purified by prep-HPLC to give desired product (5.0 mg in vial, yield: 2.6%, confirmed by ¹H NMR, HPLC and LC-MS).

HPLC purity: 98% @ 254 nm; 95% @ 214 nm. LC-MS: [M+H]⁺ = 348.1, R.T. = 4.04 min. ¹H NMR (400 MHz, DMSO-*d₆*) δ 8.93 (brs, 1H), 6.90 (d, *J =* 4.8 Hz, 1H), 6.52 (s, 1H), 3.82 (t, *J=* 4.4 Hz, 4H), 3.47 (t, *J =* 4.4 Hz, 4H), 2.75, 2.74 (s X 2, 3H), 2.25 (s, 3H).

## Claims

1. (Currently amended) A compound having the following Formula IIB: 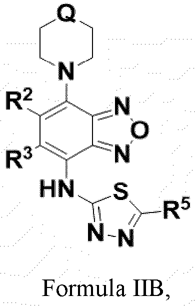
or salts thereof wherein,
Q is O;
R² is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R² is optionally substituted with one or more, the same or different, R²⁰;
R²⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R²⁰ is optionally substituted with one or more, the same or different, R²¹;
R²¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl;
R³ is selected from hydrogen, alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³ is optionally substituted with one or more, the same or different, R³⁰;
R³⁰ is selected from alkyl, alkenyl, alkanoyl, halogen, nitro, cyano, hydroxy, amino, mercapto, formyl, carboxy, carbamoyl, alkoxy, alkylthio, alkylamino, dialkylamino, alkylsulfinyl, alkylsulfonyl, arylsulfonyl, carbocyclyl, aryl, and heterocyclyl wherein R³⁰ is optionally substituted with one or more, the same or different, R³¹;
R³¹ is selected from halogen, nitro, cyano, hydroxy, trifluoromethoxy, trifluoromethyl, amino, formyl, carboxy, carbamoyl, mercapto, sulfamoyl, methyl, ethyl, propyl, tert-butyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulfinyl, ethylsulfinyl, mesyl, ethylsulfonyl, methoxycarbonyl, ethoxycarbonyl, N-methylsulfamoyl, N-ethylsulfamoyl, N,N-dimethylsulfamoyl, N,N-diethylsulfamoyl, N-methyl-N-ethylsulfamoyl, carbocyclyl, aryl, and heterocyclyl;
R⁵ is selected from amino, mercapto and alkylamino.

2. The compound of Claim 1 wherein the compound is

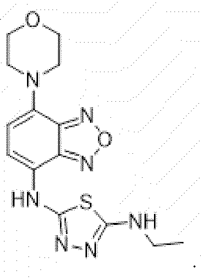

3. The compound of claim 1, wherein the compound is N2-methyl-N5-(7-morpholinobenzo[c][1,2,5]oxadiazol-4-yl)-1,3,4-thiadiazole-2,5-diamine or salt thereof
or, 5-((7-morpholinobenzo[c][1,2,5]oxadiazol-4-yl)amino)-1,3,4-thiadiazole-2-thiol or salt thereof.

4. The compound of claim 1, wherein the compound is (7-((5-(methylamino)-1,3,4-thiadiazol-2-yl)amino)-4-morpholinobenzo [c][1,2,5]oxadiazol-5-yl)methanol or salt thereof.

5. A pharmaceutical composition comprising a compound as in at least one of Claims 1-4 and a pharmaceutically acceptable excipient.

6. The pharmaceutical composition of Claim 5 wherein the pharmaceutical composition is in the form of a tablet, pill, capsule, gel, gel capsule, or cream.

7. The pharmaceutical composition of Claim 5 in the form of a sterilized pH buffered aqueous salt solution or a saline phosphate buffer between a pH of 6 to 10 or an isotonic phosphate buffered saline solution, optionally comprising a saccharide or polysaccharide.

8. The pharmaceutical composition of Claim 5 in solid form surrounded by an enteric coating.

9. The pharmaceutical composition of Claim 8, wherein the enteric coating comprises methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, or combinations thereof.

10. The pharmaceutical composition of Claim 5, wherein the pharmaceutically acceptable excipient is selected from lactose, sucrose, mannitol, triethyl citrate, dextrose, cellulose, methyl cellulose, ethyl cellulose, hydroxyl propyl cellulose, hydroxypropyl methylcellulose, carboxymethylcellulose, croscarmellose sodium, polyvinyl N-pyrrolidone, crospovidone, povidone, methyl and ethyl acrylate copolymer, polyethylene glycol, fatty acid esters of sorbitol, lauryl sulfate, gelatin, glycerin, glyceryl monooleate, silicon dioxide, titanium dioxide, talc, corn starch, carnauba wax, stearic acid, sorbic acid, magnesium stearate, calcium stearate, castor oil, mineral oil, calcium phosphate, starch, carboxymethyl ether of starch, iron oxide, triacetin, acacia gum, esters, or salts thereof.

11. The compound as in at least one of Claims 1-4 for use as a medicament for treating cancer.

12. The compound as in at least one of Claims 1-4 for use as a medicament for treating a cognitive disorder.

13. The pharmaceutical composition as in at least one of Claim 6-10 for use as a medicament for treating cancer.

14. The pharmaceutical composition as in at least one of Claim 6-10 for use as a medicament for treating a cognitive disorder.

## Patentansprüche

1. Verbindung mit der folgenden Formel IIB: 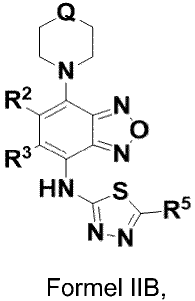
oder Salze davon, wobei
Q für O steht;
R² ausgewählt ist aus Wasserstoff, Alkyl, Alkenyl, Alkanoyl, Halogen, Nitro, Cyano, Hydroxy, Amino, Mercapto, Formyl, Carboxy, Carbamoyl, Alkoxy, Alkylthio, Alkylamino, Dialkylamino, Alkylsulfinyl, Alkylsulfonyl, Arylsulfonyl, Carbocyclyl, Aryl und Heterocyclyl, wobei R² gegebenenfalls mit einem oder mehreren, gleichen oder verschiedenen R²⁰ substituiert ist;
R²⁰ ausgewählt ist aus Alkyl, Alkenyl, Alkanoyl, Halogen, Nitro, Cyano, Hydroxy, Amino, Mercapto, Formyl, Carboxy, Carbamoyl, Alkoxy, Alkylthio, Alkylamino, Dialkylamino, Alkylsulfinyl, Alkylsulfonyl, Arylsulfonyl, Carbocyclyl, Aryl und Heterocyclyl, wobei R²⁰ gegebenenfalls mit einem oder mehreren, gleichen oder verschiedenen R²¹ substituiert ist;
R²¹ ausgewählt ist aus Halogen, Nitro, Cyano, Hydroxy, Trifluormethoxy, Trifluormethyl, Amino, Formyl, Carboxy, Carbamoyl, Mercapto, Sulfamoyl, Methyl, Ethyl, Propyl, tert-Butyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-Ethylcarbamoyl, N,N-Dimethylcarbamoyl, N,N-Diethylcarbamoyl, N-Methyl-N-ethylcarbamoyl, Methylthio, Ethylthio, Methylsulfinyl, Ethylsulfinyl, Mesyl, Ethylsulfonyl, Methoxycarbonyl, Ethoxycarbonyl, N-Methylsulfamoyl, N-Ethylsulfamoyl, N,N-Dimethylsulfamoyl, N,N-Diethylsulfamoyl, N-Methyl-N-ethylsulfamoyl, Carbocyclyl, Aryl und Heterocyclyl;
R³ ausgewählt ist aus Wasserstoff, Alkyl, Alkenyl, Alkanoyl, Halogen, Nitro, Cyano, Hydroxy, Amino, Mercapto, Formyl, Carboxy, Carbamoyl, Alkoxy, Alkylthio, Alkylamino, Dialkylamino, Alkylsulfinyl, Alkylsulfonyl, Arylsulfonyl, Carbocyclyl, Aryl und Heterocyclyl, wobei R³ gegebenenfalls mit einem oder mehreren, gleichen oder verschiedenen R³⁰ substituiert ist;
R³⁰ ausgewählt ist aus Alkyl, Alkenyl, Alkanoyl, Halogen, Nitro, Cyano, Hydroxy, Amino, Mercapto, Formyl, Carboxy, Carbamoyl, Alkoxy, Alkylthio, Alkylamino, Dialkylamino, Alkylsulfinyl, Alkylsulfonyl, Arylsulfonyl, Carbocyclyl, Aryl und Heterocyclyl, wobei R³⁰ gegebenenfalls mit einem oder mehreren, gleichen oder verschiedenen R³¹ substituiert ist;
R³¹ ausgewählt ist aus Halogen, Nitro, Cyano, Hydroxy, Trifluormethoxy, Trifluormethyl, Amino, Formyl, Carboxy, Carbamoyl, Mercapto, Sulfamoyl, Methyl, Ethyl, Propyl, tert-Butyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-Ethylcarbamoyl, N,N-Dimethylcarbamoyl, N,N-Diethylcarbamoyl, N-Methyl-N-ethylcarbamoyl, Methylthio, Ethylthio, Methylsulfinyl, Ethylsulfinyl, Mesyl, Ethylsulfonyl, Methoxycarbonyl, Ethoxycarbonyl, N-Methylsulfamoyl, N-Ethylsulfamoyl, N,N-Dimethylsulfamoyl, N,N-Diethylsulfamoyl, N-Methyl-N-ethylsulfamoyl, Carbocyclyl, Aryl und Heterocyclyl;
R⁵ ausgewählt ist aus Amino, Mercapto und Alkylamino.

2. Verbindung nach Anspruch 1, wobei die Verbindung 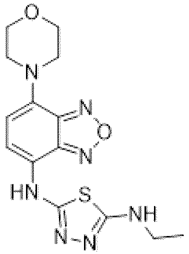 ist.

3. Verbindung nach Anspruch 1, wobei die Verbindung N2-Methyl-N5-(7-morpholinobenzo[c][1,2,5]oxadiazol-4-yl)-1,3,4-thiadiazol-2,5-diamin oder ein Salz davon ist,
oder 5-((7-Morpholinobenzo[c][1,2,5]oxadiazol-4-yl)amino)-1,3,4-thiadiazol-2-thiol oder ein Salz davon.

4. Verbindung nach Anspruch 1, wobei die Verbindung (7-((5-(Methylamino)-1,3,4-thiadiazol-2-yl)amino)-4-morpholinobenzo[c][1,2,5]oxadiazol-5-yl)methanol oder ein Salz davon ist.

5. Pharmazeutische Zusammensetzung, umfassend eine Verbindung nach mindestens einem der Ansprüche 1-4 und einen pharmazeutisch unbedenklichen Exzipienten.

6. Pharmazeutische Zusammensetzung nach Anspruch 5, wobei die pharmazeutische Zusammensetzung in Form einer Tablette, Pille, Kapsel, eines Gels, einer Gelkapsel oder einer Creme vorliegt.

7. Pharmazeutische Zusammensetzung nach Anspruch 5 in Form einer sterilisierten pH-gepufferten wässrigen Salzlösung oder eines Kochsalzphosphatpuffers mit einem pH-Wert von 6 bis 10 oder einer isotonischen phosphatgepufferten Kochsalzlösung, gegebenenfalls umfassend ein Saccharid oder Polysaccharid.

8. Pharmazeutische Zusammensetzung nach Anspruch 5 in fester Form, umgeben von einer magensaftresistenten Beschichtung.

9. Pharmazeutische Zusammensetzung nach Anspruch 8, wobei die magensaftresistente Beschichtung Methylacrylat-Methacrylsäure-Copolymere, Celluloseacetatphthalat (CAP), Celluloseacetatsuccinat, Hydroxypropylmethylcellulosephthalat, Hydroxypropylmethylcelluloseacetatsuccinat (Hypromelloseacetatsuccinat), Polyvinylacetatphthalat (PVAP), Methylmethacrylat-Methacrylsäure-Copolymere oder Kombinationen davon umfasst.

10. Pharmazeutische Zusammensetzung nach Anspruch 5, wobei der pharmazeutisch unbedenkliche Exzipient ausgewählt ist aus Lactose, Saccharose, Mannitol, Triethylcitrat, Dextrose, Cellulose, Methylcellulose, Ethylcellulose, Hydroxypropylcellulose, Hydroxypropylmethylcellulose, Carboxymethylcellulose, Croscarmellose-Natrium, Polyvinyl-N-pyrrolidon, Crospovidon, Povidon, Methyl- und Ethylacrylatcopolymer, Polyethylenglykol, Fettsäureestern von Sorbitol, Laurylsulfat, Gelatine, Glycerin, Glycerylmonooleat, Siliciumdioxid, Titandioxid, Talk, Maisstärke, Carnaubawachs, Stearinsäure, Sorbinsäure, Magnesiumstearat, Calciumstearat, Rizinusöl, Mineralöl, Calciumphosphat, Stärke, Carboxymethylether von Stärke, Eisenoxid, Triacetin, Akaziengummi, Estern oder Salzen davon.

11. Verbindung nach mindestens einem der Ansprüche 1-4 zur Verwendung als Medikament zur Behandlung von Krebs.

12. Verbindung nach mindestens einem der Ansprüche 1-4 zur Verwendung als Medikament zur Behandlung einer kognitiven Störung.

13. Pharmazeutische Zusammensetzung nach mindestens einem der Ansprüche 6-10 zur Verwendung als Medikament zur Behandlung von Krebs.

14. Pharmazeutische Zusammensetzung nach mindestens einem der Ansprüche 6-10 zur Verwendung als Medikament zur Behandlung einer kognitiven Störung.

## Revendications

1. Composé répondant à la Formule IIB suivante : 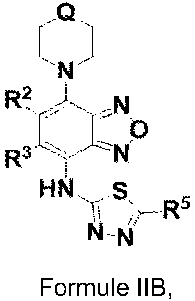
ou ses sels, dans laquelle
Q est O ;
R² est choisi parmi un hydrogène, un alkyle, un alcényle, un alcanoyle, un halogène, un nitro, un cyano, un hydroxy, un amino, un mercapto, un formyle, un carboxy, un carbamoyle, un alcoxy, un alkylthio, un alkylamino, un dialkylamino, un alkylsulfinyle, un alkylsulfonyle, un arylsulfonyle, un carbocyclyle, un aryle et un hétérocyclyle, dans lequel R² est facultativement substitué par un ou plusieurs groupes R²⁰ identiques ou différents ;
R²⁰ est choisi parmi un alkyle, un alcényle, un alcanoyle, un halogène, un nitro, un cyano, un hydroxy, un amino, un mercapto, un formyle, un carboxy, un carbamoyle, un alcoxy, un alkylthio, un alkylamino, un dialkylamino, un alkylsulfinyle, un alkylsulfonyle, un arylsulfonyle, un carbocyclyle, un aryle et un hétérocyclyle, dans lequel R²⁰ est facultativement substitué par un ou plusieurs groupes R²¹ identiques ou différents ;
R²¹ est choisi parmi un halogène, un nitro, un cyano, un hydroxy, un trifluorométhoxy, un trifluorométhyle, un amino, un formyle, un carboxy, un carbamoyle, un mercapto, un sulfamoyle, un méthyle, un éthyle, un propyle, un tert-butyle, un méthoxy, un éthoxy, un acétyle, un acétoxy, un méthylamino, un éthylamino, un diméthylamino, un diéthylamino, un N-méthyl-N-éthylamino, un acétylamino, un N-méthylcarbamoyle, un N-éthylcarbamoyle, un N,N-diméthylcarbamoyle, un N,N-diéthylcarbamoyle, un N-méthyl-N-éthylcarbamoyle, un méthylthio, un éthylthio, un méthylsulfinyle, un éthylsulfinyle, un mésyle, un éthylsulfonyle, un méthoxycarbonyle, un éthoxycarbonyle, un N-méthylsulfamoyle, un N-éthylsulfamoyle, un N,N-diméthylsulfamoyle, un N,N-diéthylsulfamoyle, un N-méthyl-N-éthylsulfamoyle, un carbocyclyle, un aryle et un hétérocyclyle ;
R³ est choisi parmi un hydrogène, un alkyle, un alcényle, un alcanoyle, un halogène, un nitro, un cyano, un hydroxy, un amino, un mercapto, un formyle, un carboxy, un carbamoyle, un alcoxy, un alkylthio, un alkylamino, un dialkylamino, un alkylsulfinyle, un alkylsulfonyle, un arylsulfonyle, un carbocyclyle, un aryle et un hétérocyclyle, dans lequel R³ est facultativement substitué par un ou plusieurs groupes R³⁰ identiques ou différents ;
R³⁰ est choisi parmi un alkyle, un alcényle, un alcanoyle, un halogène, un nitro, un cyano, un hydroxy, un amino, un mercapto, un formyle, un carboxy, un carbamoyle, un alcoxy, un alkylthio, un alkylamino, un dialkylamino, un alkylsulfinyle, un alkylsulfonyle, un arylsulfonyle, un carbocyclyle, un aryle et un hétérocyclyle, dans lequel R³⁰ est facultativement substitué par un ou plusieurs groupes R³¹ identiques ou différents ;
R³¹ est choisi parmi un halogène, un nitro, un cyano, un hydroxy, un trifluorométhoxy, un trifluorométhyle, un amino, un formyle, un carboxy, un carbamoyle, un mercapto, un sulfamoyle, un méthyle, un éthyle, un propyle, un tert-butyle, un méthoxy, un éthoxy, un acétyle, un acétoxy, un méthylamino, un éthylamino, un diméthylamino, un diéthylamino, un N-méthyl-N-éthylamino, un acétylamino, un N-méthylcarbamoyle, un N-éthylcarbamoyle, un N,N-diméthylcarbamoyle, un N,N-diéthylcarbamoyle, un N-méthyl-N-éthylcarbamoyle, un méthylthio, un éthylthio, un méthylsulfinyle, un éthylsulfinyle, un mésyle, un éthylsulfonyle, un méthoxycarbonyle, un éthoxycarbonyle, un N-méthylsulfamoyle, un N-éthylsulfamoyle, un N,N-diméthylsulfamoyle, un N,N-diéthylsulfamoyle, un N-méthyl-N-éthylsulfamoyle, un carbocyclyle, un aryle et un hétérocyclyle ;
R⁵ est choisi parmi un amino, un mercapto et un alkylamino.

2. Composé selon la revendication 1, dans lequel le composé est

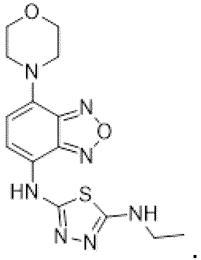

3. Composé selon la revendication 1, dans lequel le composé est la N2-méthyl-N5-(7-morpholinobenzo[c][1,2,5]oxadiazol-4-yl)-1,3,4-thiadiazole-2,5-diamine ou un sel de celle-ci,
ou le 5-((7-morpholinobenzo[c][1,2,5]oxadiazol-4-yl)amino)-1,3,4-thiadiazole-2-thiol ou un sel de celui-ci.

4. Composé selon la revendication 1, dans lequel le composé est le (7-((5-(méthylamino)-1,3,4-thiadiazol-2-yl)amino)-4-morpholinobenzo[c][1,2,5]oxadiazol-5-yl)méthanol ou un sel de celui-ci.

5. Composition pharmaceutique comprenant un composé selon au moins l'une des revendications 1 à 4 et un excipient pharmaceutiquement acceptable.

6. Composition pharmaceutique selon la revendication 5, dans laquelle la composition pharmaceutique est sous la forme d'un comprimé, d'une pilule, d'une capsule, d'un gel, d'une gélule ou d'une crème.

7. Composition pharmaceutique selon la revendication 5, sous la forme d'une solution aqueuse saline stérilisée et tamponnée en pH ou d'un tampon phosphate salin ayant un pH compris entre 6 et 10 ou d'une solution saline isotonique tamponnée au phosphate, comprenant facultativement un saccharide ou un polysaccharide.

8. Composition pharmaceutique selon la revendication 5, sous forme solide entourée d'un enrobage entérique.

9. Composition pharmaceutique selon la revendication 8, dans laquelle l'enrobage entérique comprend des copolymères d'acrylate de méthyle-acide méthacrylique, du phtalate d'acétate de cellulose (CAP), du succinate d'acétate de cellulose, du phtalate d'hydroxypropylméthylcellulose, du succinate d'acétate d'hydroxypropylméthylcellulose (succinate d'acétate d'hypromellose), du phtalate d'acétate de polyvinyle (PVAP), des copolymères de méthacrylate de méthyle-acide méthacrylique, ou des combinaisons de ceux-ci.

10. Composition pharmaceutique selon la revendication 5, dans laquelle l'excipient pharmaceutiquement acceptable est choisi parmi le lactose, le saccharose, le mannitol, le citrate de triéthyle, le dextrose, la cellulose, la méthylcellulose, l'éthylcellulose, l'hydroxypropylcellulose, l'hydroxypropylméthylcellulose, la carboxyméthylcellulose, la croscarmellose sodique, la polyvinyl-N-pyrrolidone, la crospovidone, la povidone, un copolymère d'acrylates de méthyle et d'éthyle, le polyéthylène glycol, des esters d'acide gras de sorbitol, le laurylsulfate, la gélatine, la glycérine, le monooléate de glycéryle, le dioxyde de silicium, le dioxyde de titane, le talc, l'amidon de maïs, la cire de carnauba, l'acide stéarique, l'acide sorbique, le stéarate de magnésium, le stéarate de calcium, l'huile de ricin, l'huile minérale, le phosphate de calcium, l'amidon, l'éther carboxyméthylique d'amidon, l'oxyde de fer, la triacétine, la gomme d'acacia, des esters, ou des sels de ceux-ci.

11. Composé selon au moins l'une des revendications 1 à 4 pour une utilisation en tant que médicament pour le traitement du cancer.

12. Composé selon au moins l'une des revendications 1 à 4 pour une utilisation en tant que médicament pour le traitement d'un trouble cognitif.

13. Composition pharmaceutique selon au moins l'une des revendications 6 à 10 pour une utilisation en tant que médicament pour le traitement du cancer.

14. Composition pharmaceutique selon au moins l'une des revendications 6 à 10 pour une utilisation en tant que médicament pour le traitement d'un trouble cognitif.
